# EUROPÄISCHE PATENTSCHRIFT

(11) **EP 1 754 468 B1**
(45) Veröffentlichungstag und Bekanntmachung des Hinweises auf die Patenterteilung: **08.04.2009**
(21) Anmeldenummer: 06016885.3
(22) Anmeldetag: 12.08.2006
(51) Int. Cl.: A61K 8/89, A61K 8/898, A61Q 5/00, C08G 77/388, C08G 77/04

(54) **Verwendung von quaternären Polysiloxanen in Reinigungs- und Pflegemitteln**
Use of quaternary polysiloxanes in wash and personal care products
Utilisation de siloxanes quaternaires dans des produits hygiéniques

(30) Priorität: 20.08.2005 DE 102005039511
(43) Veröffentlichungstag der Anmeldung: 21.02.2007
(73) Patentinhaber: Clariant Produkte (Deutschland) GmbH, 65929 Frankfurt am Main (DE)
(72) Erfinder: Meder, Markus, Dr., 65779 Kelkheim (DE); Haala, Sabine, 63457 Hanau (DE); Müller, Carsten, 60486 Frankfurt am Main (DE)
(74) Vertreter: Paczkowski, Marcus

(56) Entgegenhaltungen:
- WO-A-03/080007
- WO-A-20/05035628
- DE-A1- 3 340 708

## Beschreibung

Die Erfindung betrifft die Verwendung von quaternäre Ammoniumgruppen enthaltenden Polysiloxanverbindungen zur Reinigung und Pflege von keratinhaltigen Substraten, insbesondere von menschlichen Haaren.

Es ist bekannt, dass polysiloxanbasierte quaternäre Verbindungen (Quats) mit Vorteil in Pflegemittel für keratinhaltige Substrate, insbesondere für Haarpflegemittel genutzt werden können. In EP-A 282720 und DE-A 37 19 086 werden quaternäre Polysiloxane und deren Verwendung in konditionierenden Haarpflegeprodukten beansprucht. Diese quaternären Polysiloxane zeigen zwar meist eine weich machende Wirkung, verbessern die Kämmbarkeit und reduzieren die elektrostatische Aufladung der Haare, sie sind aber bezüglich der Farbstabilisierung gefärbter Haare unzureichend.

In WO 2005/035 628 werden mehrfach quaternäre Polysiloxane und deren Verwendung als weichmachende Komponente in der Textilindustrie bei der Herstellung der Textilien beschrieben.

WO 03/080007 beschreibt Zusammensetzungen enthaltend Polyorganosiloxane mit mindestens einer quarternären Gruppe. DE 3 340 708 offenbart polyquaternäre Polysiloxan Polymere und diese enthaltende Kosmetische Mittel.

Es bestand die Aufgabe, Substanzen für die Behandlung keratinischer Fasern zur Verfügung zu stellen, die eine gute Konditionierung, z.B. von Haut, Haaren und Textilien bewirken, die Farbbrillianz und den Glanz verbessern, leicht in Formulierungen eingearbeitet werden können, ein möglichst klares Erscheinungsbild ergeben und die eine konditionierende Wirkung zeigen. Darüber hinaus sollen die Mittel eine gute "Substantivität" aufweisen, d.h. für getönte oder gefärbte Haare eine Verbesserung des Farbaufziehverhaltens und eine Erhöhung der Farbstabilität und Formbeständigkeit bewirken. Insbesondere besteht der Bedarf an Haarpflege- und Reinigungsmittel, die das Auswaschen der Farbe von getönten und gefärbten Haaren verhindern und die Haare bei Hitze- und Sonneneinwirkung schonen.

Es wurde überraschend gefunden, dass diese Aufgabe gelöst wird durch Polysiloxane, die mehrere quaternäre Ammoniumgruppen enthalten.

Gegenstand der Erfindung sind somit Mittel zum Reinigen und Pflegen keratinischer Fasern, insbesondere von menschlichen Haaren, enthaltend mehrfach quaternäre Polysiloxane der Formel (S1) 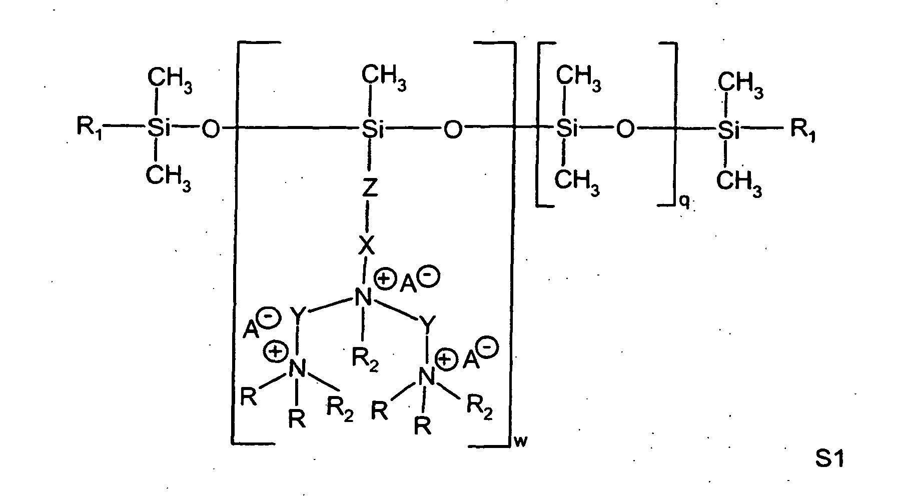 worin
die Summe aus (q + w) einen Bereich von 10 bis 1500, vorzugsweise von 15 bis 600 aufweist, und das Verhältnis q/w einen Bereich von 5 bis 600, vorzugsweise von 10 bis 400 aufweist,
- R: C₁-C₄-Alkyl, linear oder verzweigt, bedeutet,
- R₁: Wasserstoff, C₁-C₃-Alkyl oder C₁-C₃-Alkoxy,
- R₂: C₁-C₇Alkyl oder Benzyl,
- X: eine direkte Bindung,
oder 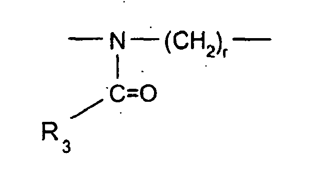 worin
- r: eine ganze Zahl von 1 bis 4, und
- R₃: C₁-C₇-Alkyl oder -NH-C₁-C₇Alkyl bedeutet,
oder 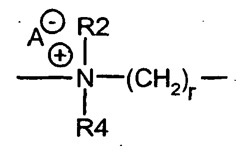 worin
- R₂ und r: wie vorher definiert sind und
- R₄: C₁-C₃-Alkyl bedeutet,
oder 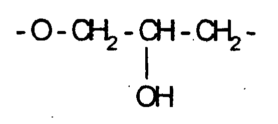 bedeutet,
Y 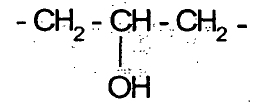 oder

-(CH₂)ₓ-

bedeutet,
- ·: worin x eine ganze Zahl von 1 bis 4 darstellt,
- Z: C₂-C₄-Alkylen, linear oder verzweigt, ist,
- A⁻: CH₃OSO₃⁻, Chlorid, Bromid, lodid oder Tosylsulfat⁻ darstellt,
oder der Formel (S2) 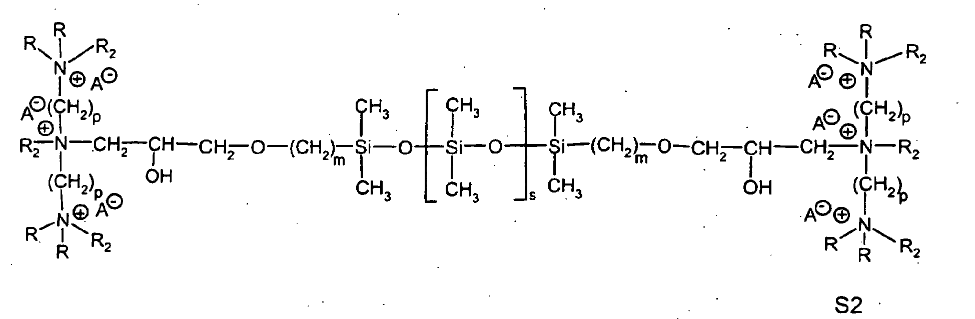 worin
R, R₂ und A⁻ die gleiche Bedeutung wie in Formel (S1) haben,
m eine ganze Zahl von 1 bis 4,
p eine ganze Zahl von 1 bis 4, und
s eine Zahl im Bereich von 5 bis 1500, vorzugsweise von 10 bis 600 bedeuten. Bevorzugt sind Verbindungen worin
R Methyl, Ethyl, oder Propyl,
R₁ H, Methyl, -OCH₃ oder -OC₂H₅,
R₂ Methyl oder Benzyl,
R₃ Methyl oder -NH-C₄H₉,
R₄ Methyl,
A⁻ CH₃OSO₃⁻ oder Chlorid,
- Z: C₃-Alkylen, linear oder verzweigt, ist,
- m: 3,
- p: 3
- s: 10 bis 600,
- r: 2 und
- x: 3 bedeutet.

Ganz besonders geeignet sind Polysiloxane mit folgenden Struktureinheiten:

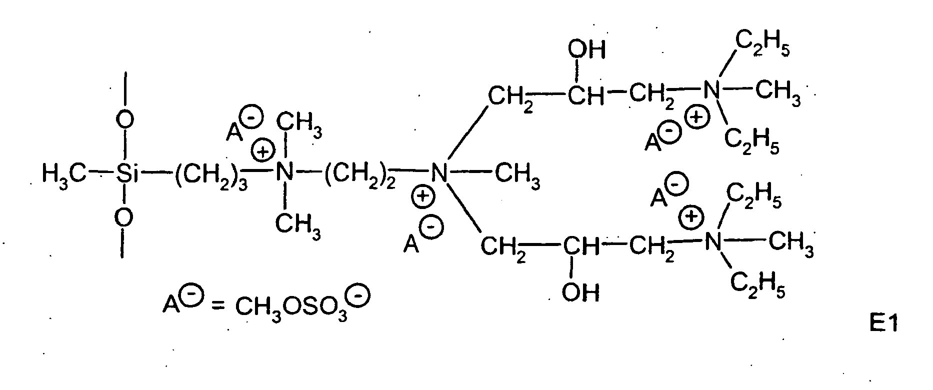

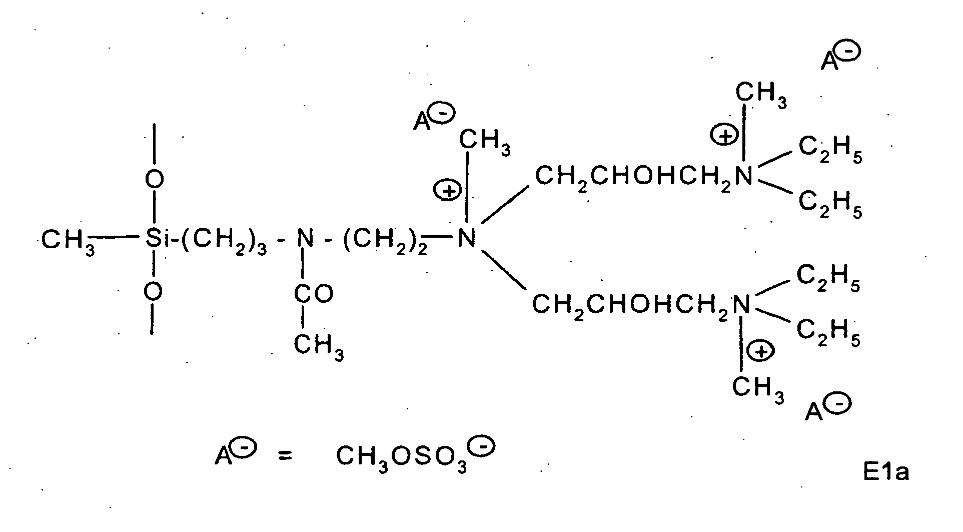

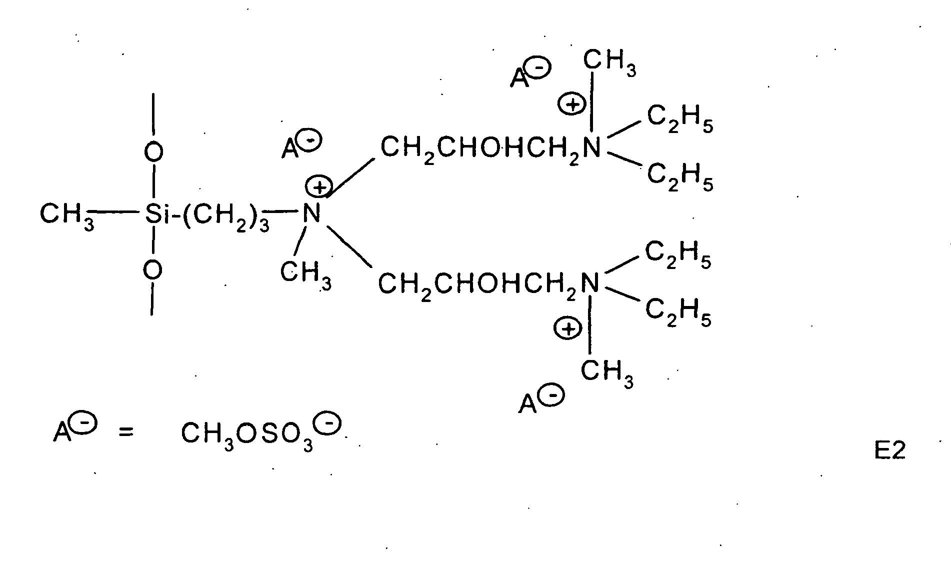

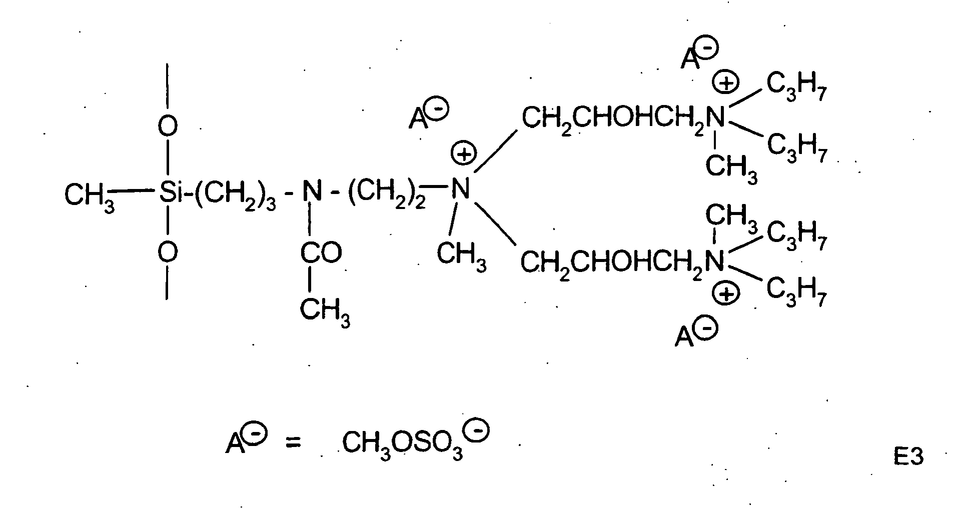

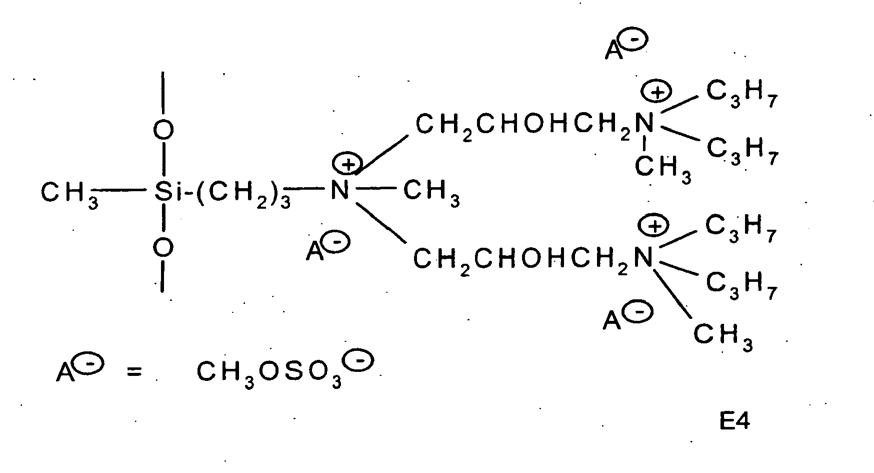

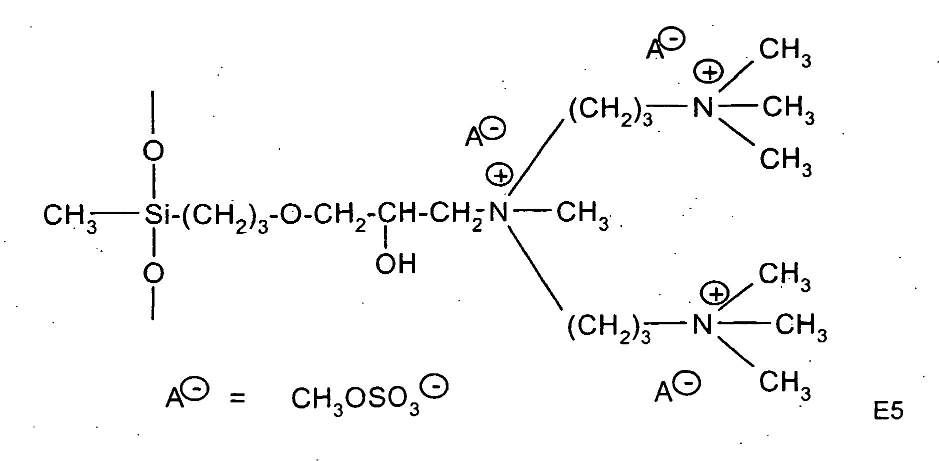

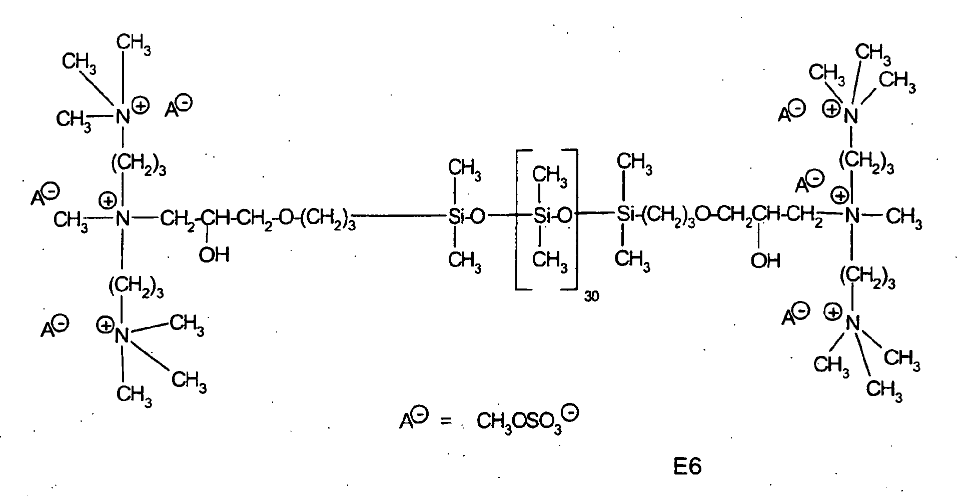

Außerordentlich bevorzugt sind Polysiloxane mit den Struktureinheiten E1.

Die Herstellung der vorgenannten Polysiloxane erfolgt in folgender Weise.

Die Verbindungen der Formel (S1), in denen Y 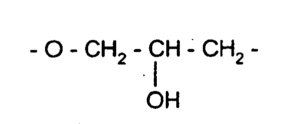 und X 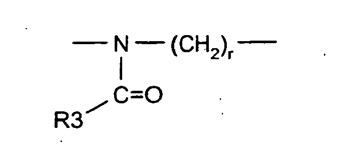 bedeuten, lassen sich herstellen durch Umsetzung von 3-(2-Aminoalkylamino)-alkyldialkoxymethylsilan mit Glycidyldialkylamin zum entsprechenden Silan, und anschließender Umsetzung der gebildeten Silane mit a) Polydimethylsiloxandiol oder mit Octamethylcyclotetrasiloxan, und mit b) Tetraalkyl- oder Arylalkylammoniumhydroxid (z.B. Benzyltrimethyl-, Tetramethyl- oder Tetrabutyl-ammoniumhydroxid) zu Polysiloxanen mit anschließender Quaternierung zu den mehrfach quaternären Siloxanen. Bevorzugte Ausgangssubstanzen sind 3-(2-Aminoethylamino)-propyl-dimethoxymethylsilan, 3-(2-Aminoethylamino)-propyl-diethoxymethylsilan und Glycidyldimethylamin, Glycidyldiethylamin und Glycidyldipropylamin. Beispiele hierfür sind die Endprodukte E1a und E3.

Zur Quaternierung können an sich bekannte Quaternierungsmittel eingesetzt werden, wie sie zur Quaternierung von tertiären Aminogruppen eingesetzt werden, z.B. Alkylhalogenide oder Dialkylsulfate, z.B. Dimethylsulfat, Diethylsulfat oder Methyl- oder Ethylchlorid oder -bromid, oder Benzylchlorid. Dabei ist es von Vorteil, Benzylchlorid oder vorzugsweise ein Dialkylsulfat dafür zu verwenden. Dabei entsteht das entsprechende Gegenion (besonders Chlorid- oder Alkylsulfation) zum jeweiligen gebildeten quaternären Ammoniumion. Besonders bevorzugt ist Dimethylsulfat.

Die Polysiloxane der Formel (S1), in denen Y 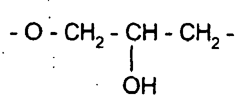 und X eine direkte Bindung bedeutet, lassen sich herstellen durch Umsetzung von 3-Aminoalkyl-dialkoxy-methylsilan mit Glycidyldialkylamin (herstellbar durch Reaktion von Dialkylamin mit Epichlorhydrin) zum entsprechenden Silan, und anschließender Umsetzung der gebildeten Silane mit a) Polydimethylsiloxandiol oder mit Octamethylcyclotetrasiloxan, und mit b) Tetraalkyl- oder Arylalkylammoniumhydroxid (z.B. Benzyltrimethyl-, Tetramethyl- oder Tetrabutyl-ammoniumhydroxid) zu Polysiloxanen mit anschließender Quaternierung zu den mehrfach quaternierten Siloxanen.

Bevorzugte Ausgangssubstanzen sind 3-Aminopropyl-diethoxy-methylsilan, 3-Aminopropyl-dimethoxy-methylsilan und Glycidyldimethylamin, Glycidyldiethylamin und Glycidyldipropylamin. Beispiele hierfür sind die Struktureinheiten E2 und E4.

Die Polysiloxane der Formel (S1) in denen Y -(CH₂)ₓ- und X 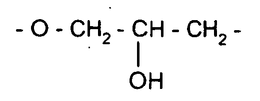 bedeutet, lassen sich herstellen durch Umsetzung von N'-[3-(Dialkylamino)alkyl]-N,N-dialkylalkan-1,3-diamin mit Dialkoxy-(3-glycidyloxyalkyl)-methylsilan und anschließender Reaktion mit Polydimethylsiloxandiol oder mit Octamethylcyclotetrasiloxan mit nachfolgender Quaternierung.

Bevorzugte Ausgangssubstanzen sind N'-[3-(Dimethylamino)propyl]-N,N-dimethylpropan-1,3-diamin, Diethoxy-(3-glycidyloxypropyl)-methylsilan und Dimethoxy-(3-glycidyloxypropyl)-methylsilan. Als Beispiel hierfür sei die Struktureinheit E5 genannt.

Die Verbindungen der Formel (S2) lassen sich beispielsweise herstellen durch Umsetzung von Octamethylcyclotetrasiloxan mit 1,1,3,3-Tetraalkyldisiloxan, vorzugsweise 1,1,3,3-Tetramethyldisiloxan, Umsetzung des Reaktionsproduktes mit einem Allylglycidylether und einem Hydrosilylierungskatalysator, Umsetzung dieses Reaktionsproduktes mit N,N,N',N'-Tetraalkyl-dialkylen-triamin, vorzugsweise N,N,N',N'-Tetramethyl-dipropylen-triamin, zum Polysiloxan, und nachfolgender Quaternierung. Als Beispiel hierfür sei die Struktur E6 genannt.

Statt Octamethylcyclotetrasiloxan können auch Penta- oder Hexamethylcyclo-tetrasiloxan oder Gemische daraus verwendet werden.

Die erfindungsgemäß eingesetzten mehrfach quaternären Polysiloxane zeigen eine hervorragende Substantivität gegenüber keratinischen Fasern, sowie gute konditionierende und farberhaltende bis farbvertiefende Effekte, insbesondere gegenüber Haaren. Besonders vorteilhaft ist, dass Haarpflege- und -reinigungsmittel, enthaltend oben definierte mehrfach quaternären Polysiloxane, ein Auswaschen der Farben von getönten und gefärbten Haaren verhindern oder minimieren.

Das Farbaufziehverhalten von Haarfärbemitteln kann durch die erfindungsgemäß eingesetzten mehrfach quaternären Polysiloxane verbessert werden. In Haarstylingmitteln ist zudem eine volumen- und glanzgebende Wirkung der mehrfach quaternären Polysiloxane signifikant. Vorteilhaft sind außerdem die gute Wasserlöslichkeit, ein günstiges Viskositätsverhalten, die gute Einarbeitbarkeit, sowie ein klares Erscheinungsbild der erfindungsgemäß eingesetzten mehrfach quaternären Polysiloxane. Darüber hinaus sind sie unempfindlich gegenüber UV- und IR-Strahlung. Sie sind somit wertvolle Bestandteile für Haarpflege- und Haarreinigungsmittel, sowie Haarfärbemittel.

In einer bevorzugten Ausführungsform der Erfindung liegen die kosmetischen oder pharmazeutischen Mittel in wässriger, wässrig-alkoholischer, alkoholischer oder wässrig-tensidischer Form vor oder sie stellen Mittel auf Basis von Öl, dar oder liegen als Emulsion, Suspension oder Dispersion vor und zwar in Form von Fluids, Schäumen, Sprays, Gelen, Mousse, Lotionen oder Cremes.

Bei den Emulsionen kann es sich sowohl um Wasser-in-Öl-Emulsionen als auch Öl-in-Wasser-Emulsionen, Mikroemulsionen, Nanoemulsionen und multiple Emulsionen handeln. Die Herstellung der Emulsionen kann in bekannter Weise, d.h. beispielsweise durch Kalt-, Heiß-, Heiß/Kalt- bzw. PIT-Emulgierung erfolgen.

Die erfindungsgemäß in kosmetischen Mitteln eingesetzten mehrfach quaternären Polysiloxane haben eine ausgeprägte Hydrophilie, die durch Einbau entsprechender Gruppen und Substituenten modifiziert werden kann.

Gute Substantivität, konditionierende Wirkung, sowie glanz- und volumengebende Effekte der oben beschriebenen mehrfach quaternären Polysiloxane macht man sich erfindungsgemäß zur Herstellung von Haarbehandlungsmitteln, vorzugsweise Schampoos, Haarconditioner, Haarkuren, Stylingmittel, Haarspülungen, Volumenspray, Styling-Fluid, Haarschaum, Haargel, Festiger, Haarspray, Mousse, Haarölen und Spitzenfluids zu Nutze.

Die oben beschriebenen mehrfach quaternären Polysiloxane verbessern das Farbaufziehverhalten von Haarfärbemittel und sind somit wertvolle Bestandteile in Haartönungs- und -färbemitteln. Gleichzeitig verbessern sie zusätzlich als Farbschutzadditive die Haltbarkeit von Haartönungen oder permanten Haarfärbemitteln und erhöhen signifikant den Glanz, insbesondere der gefärbten Haare.

Weiterhin Gegenstand der Erfindung ist somit auch die Verwendung eines erfindungsgemäßen kosmetischen oder pharmazeutischen Mittels zum Schutz und zur Erhaltung der Farbe in gefärbten Keratinfasern, vorzugsweise in gefärbtem menschlichem Haar. Vorzugsweise enthält das erfindungsgemäße Mittel bei dieser Verwendung von 0,01 bis 10 Gew.-% bezogen auf das fertige Mittel an quaternärem Polysiloxan .

In bevorzugten Ausführungsformen enthalten die Haarpflege- und Reinigungsmittel UV-Filter.

In einer weiteren bevorzugten Ausführungsform der Erfindung handelt es sich bei den kosmetischen und pharmazeutischen Mitteln um tensidfreie Mittel, um tensidfreie Emulsionen, Gele, Sprays, Sprühschäume, Mousse oder Fluids.

In einer weiteren bevorzugten Ausführungsform der Erfindung handelt es sich bei den kosmetischen oder pharmazeutischen Mitteln um Additive für Dauerwellenmittel, insbesondere um Conditioner zur Nachbehandlung.

Die erfindungsgemäßen kosmetischen oder pharmazeutischen Mittel auf wässriger oder wässrig-alkoholischer Basis enthalten mehrfach quaternäre Polysiloxane vorzugsweise in den Gewichtsmengen von 0,01 bis 30 %, besonders bevorzugt von 0,2 bis 10 %, insbesondere bevorzugt von 0,5 bis 2 %, bezogen auf die fertigen Mittel.

Die erfindungsgemäßen kosmetischen oder pharmazeutischen Mittel in Form einer Emulsion enthalten mehrfach quaternäre Polysiloxane vorzugsweise in Gewichtsmengen von 0,01 bis 30 %, besonders bevorzugt von 0,05 bis 10 % und insbesondere bevorzugt von 0,1 bis 5 %, bezogen auf das fertige Mittel.

In einer weiteren bevorzugten Ausführungsform sind die erfindungsgemäßen Mittel Öl-in-Wasser Emulsionen mit einem Wasseranteil von 5 bis 95 Gew.-%, bevorzugt 15 bis 75 Gew.-%, besonders bevorzugt 25 bis 85 Gew.-%.

Für die erfindungsgemäßen Mittel auf wässrig-alkoholischer oder alkoholischer Basis kommen alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol oder Glycerin sowie Alkylenglykole, insbesondere Propylen-, Butylen- oder Hexylenglykol, und Mischungen aus den genannten Alkoholen. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und von Polyethylenglykol mit einer relativen Molekülmasse zwischen 400 und 600 bevorzugt.

Die erfindungsgemäßen Mittel können folgende Öle enthalten: Kohlenwasserstofföle mit linearen oder verzweigten, gesättigten oder ungesättigten C₇-C₄₀-Kohlenstoffketten, beispielsweise Dodecan, Isododecan, Cholesterol, hydrierte Polyisobutylene, Docosane, Hexadecan, Isohexadecan, Paraffine und Isoparaffine, aber auch Triglyceride tierischen und pflanzlichen Ursprungs, beispielweise Rindertalg, Schweineschmalz, Gänseschmalz, Perhydrosqualen, Lanolin, Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Olivenöl, Erdnussöl, Rapsöl und Kokosnussöl und synthetische Öle wie Purcellinöl, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Alkoholester von C₁-C₁₀-Carbonsäuren oder C₂-C₃₀-Dicarbonsäuren, Ester wie Dioctyladipat, Diisopropyl dimer dilineloat; Propylenglycole/-dicaprilat oder Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle; Monoglyceride von C₁-C₃₀-Carbonsäuren, Diglyceride von C₁-C₃₀-Carbonsäuren, Triglyceride von C₁-C₃₀-Carbonsäuren, beispielsweise Triglyceride der Capryl/Caprinsäuren, Ethylenglykolmonoester von C₁-C₃₀-Carbonsäuren, Ethylenglycoldiester von C₁-C₃₀-Carbonsäuren, Propylenglycolmonoester von C₁-C₃₀-Carbonsäuren, Propylenglycoldiester von C₁-C₃₀-Carbonsäuren, sowie propoxilierte und ethoxilierte Derivate der oben genannten Verbindungsklassen. Die Carbonsäuren können lineare oder verzweigte Alkylgruppen oder aromatische Gruppen enthalten. Beispielhaft genannt seien Diisopropylsebacat, Diisopropyladipate, Isopropylmyristat, Isopropylpalmitat, Myristylpropionat, Ethylenglycoldistearat, 2-Ethylhexylpalmitat, Isodecylneopentanoat, Di-2-Ethylhexylmaleat, Cetylpalmitat, Myristylmyristat, Stearylstearat, Cetylstearat, Behenylbehenat, Dioctylmaleate, Dioctylsebacat, Cetyloctanoat, Diisopropyl dilinoleate, Caprylic/ Capryltriglycerid, PEG-6-Caprylic/Capryltriglycerid, PEG-8- Caprylic/ Capryltriglycerid, Cetylricinoleat, Cholesterolhydroxystearat, Cholesterolisostearat, C₁-C₃₀-Monoester und Polyester von Glycerin, beispielsweise Glyceryltribehenat, Glycerylstearat, Glycerylpalmitat, Glyceryldistearat, Glyceryldipalmitat, C₁-C₃₀-Carbonsäuremonoester und Polyester von Zucker, beispielsweise Glucosetetraoleat, Glucosetetraester der Sojaölfettsäure, Mannosetetraester der Sojaölfettsäure, Galactosetetraester der Ölsäure, Arabinosetetraester der Linolsäure, Xylosetetralinoleat, Galactosepentaoleat, Sorbitoltetraoleat, Sorbitolhexaester der ungesättigten Sojaölfettsäure, Xylitolpentaoleat, Sucrosetetraoleat, Sucrosepentaoleat, Sucrosehexaoleat, Sucroseheptaoleat, Sucroseoleat.

An Silikonölen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500, beispielsweise die unter den Handelsnamen VICASIL (General Electric Company), DOW CORNING 200, DOW CORNING 225, DOW CORNING 200 (Dow Corning Corporation), erhältlichen Dimethicone, Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen kosmetischen und pharmazeutischen Mittel enthalten oben genannte Silikonöle in den Gewichtsmengen von 0,5 % bis 15 %, bevorzugt 1 % bis 10 %, besonders bevorzugt 1,5 % bis 5 %.

Die erfindungsgemäßen Haarfärbe- und -tönungsmittel enthalten vorzugsweise Direktfarbstoffe und/oder Oxidationsfarbstoffvorstufen in den üblichen pH-Bereichen. An Direktfarbstoffen in Betracht kommen vorzugsweise Nitroanilinderivate, wie 1-[(2-Hydroxyethyl)-amino]-2-nitrobenzol (Velsol^{®} Yellow 2), 4-Hydroxypropylamino-3-Nitrophenol (Velsol Red BN), 3-Nitro-p-Hydroxyethylaminophenol (Velsol Red 54), 4-Hydroxyethylamino-3-Nitroanilin (Velsol Red 3), N,N'-Bis-(Hydroxyethyl)-2-Nitro-p-Phenylendiamine (Velsol Violet BS), N,N',N'-Tris-(Hydroxyethyl)-2-Nitro-p-Phenylendiamine (Velsol Blue 2), 4-(2'-Hydroxyethyl)-amino-3-nitro-toluol, 4-(2'-Hydroxyethyl)-amino-3-nitro-benzylalkohol, 4-(2'-Hydroxyethyl)-amino-3-nitro-1-trifluormethyl-benzol, 4-(2',3'-Dihydroxypropyl)-amino-3-nitro-chlorbenzol, 4-(2'-Hydroxyethyl)-amino-3-nitro-brombenzol und 4-(2',3'-Dihydroxypropyl)-amino-3-nitro-brombenzol, Nitrobenzolderivate, beispielsweise 2-Amino-4-nitro-phenol, Pikraminsäure, 1-[(2'-Hydroxyethyl)-amino]-2-amino-4-nitrobenzol, 2-Nitro-4-[(2'-hydroxyethyl)amino]-anilin, 4-Bis-[(2'-hydroxyethyl)amino]-1-methylamino-2-nitro-benzol, 2,5-Bis-[(2'-hydroxyethyl)-amino]-nitrobenzol, 2-(2'-hydroxyethyl)-amino-4,6-dinitro-phenol, 1-Amino-4-(2',3'-dihydroxypropyl)-amino-2-nitro-5-chlor-benzol, aber auch Triphenylmethanfarbstoffe, wie zum Beispiel Basic Violet 1 (C.I. 42535), Azofarbstoffe, wie zum Beispiel Acid Brown 4 (C.I. 14805), Anthrachinonfarbstoffe, wie zum Beispiel Disperse Blue 23 (C.I. 61545), Disperse Violet 4 (C.I.61105), 1,4,5,8-Tetraaminoanthrachinon und 1,4-Diaminoanthrachinon und weitere direktziehende Farbstoffe.

An Oxidationsfarbstoffvorstufen stehen vorzugsweise zur Verfügung p-Phenylendiamine und p-Aminophenole oder deren Derivate wie beispielsweise p-Toluylendiamin, p-Phenylendiamin, p-Aminophenol, die zum Zweck der Nuancierung der Färbung mit so genannten Modifiern oder Kupplern, wie zum Beispiel m-Phenylendiamin, Resorcin, m-Aminophenol und deren Derivaten kombiniert werden.

Als Oxidationsmittel zur Entwicklung der Haarfärbungen kommen vorzugsweise Hydrogenperoxyd und dessen Additionsverbindungen in Betracht.

Die erfindungsgemäß eingesetzten mehrfach quaternären Polysiloxane sind sehr gut kompatibel mit perlglanzgebenden Komponenten. Die erfindungsgemäßen Haarbehandlungsmittel können somit vorteilhafter Weise perlglanzgebende Verbindungen enthalten, beispielsweise Fettsäuremonoalkanolamide, Fettsäuredialkanolamide, Monoester oder Diester von Alkylenglycol, insbesondere Ethylenglykol und/oder Propylenglykol oder dessen Oligomere mit höheren Fettsäuren, z.B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art, bevorzugt Ethylenglycoldistearat und Polyethylenglycoldistearat mit ca. 3 Glykoleinheiten.

Die erfindungsgemäßen Haarbehandlungsmittel enthalten bevorzugt 0,1 bis 15, besonders bevorzugt 1 bis 10 Gew.-% an perlglanzgebenden Verbindungen.

Glitter- und Glanzeffekte der erfindungsgemäßen Mittel lassen sich vorzugsweise durch Zugabe von Glimmer, colorierte Polyacrylester und Glimmer, Glimmer-Eisenoxid, Glimmer-Titanoxid und durch Pigmente erzeugen. Als Pigmente eignen sich Metalloxide, beispielsweise Eisenoxide, Titanoxid, Ultramarinblau, sowie mit kationischen Coatinghüllen modifizierte Pigmente, wie in WO 00/12053 beschrieben.

Die erfindungsgemäßen Haarbehandlungsmittel können als weitere Hilfs- und Zusatzstoffe Tenside, Emulgatoren, kationische Polymere, Verdicker, Filmbildner, antimikrobielle Wirkstoffe, Antioxidation, Pigmente/Mikropigmente, Gelierungsmittel, sowie weitere in der Kosmetik gebräuchliche Zusätze, wie z.B. Überfettungsmittel, feuchtigkeitsspendende Mittel, Silikone, Stabilisatoren, weitere Konditioniermittel, Glycerin, Konservierungsmittel, Perlglanzmittel, Farbstoffe, Duft- und Parfümöle, Lösungsmittel, Hydrotrope, Trübungsmittel, Fettalkohole, Antischuppenmittel, Vitamine, Bisabolol^{®}, Allantoin^{®}, Phytantriol^{®}, Panthenol^{®}, AHA-Säuren, Pflanzenextrakte, beispielsweise Aloe Vera und Proteine enthalten.

Als anionische waschaktive Substanzen seien vorzugsweise genannt: C₁₀-C₂₀-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäürehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäure-methyltauride, Fettsäuresarkosinate, Sulforicinoleate, Amphoacetate- oder -glycinate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside beträgt bevorzugt 1 bis 30 Gew.-%, besonders bevorzugt 5 bis 25 Gew.-%, insbesondere bevorzugt 10 bis 22 Gew.-%, bezogen auf die fertigen Mittel.

Geeignete kationische Tenside sind beispielsweise quartäre Ammonium-Salze wie Di-(C₁₀-C₂₄-Alkyl)-dimethyl-ammonium-chlorid oder -bromid, vorzugsweise Di-(C₁₂-C₁₈-Alkyl)-dimethyl-ammonium-chlorid oder -bromid; C₁₀-C₂₄-Alkyl-dimethyl-ethylammonium-chlorid oder -bromid; C₁₀-C₂₄-Alkyl-trimethyl-ammonium-chlorid oder -bromid, vorzugsweise Cetyltrimethyl-ammonium-chlorid oder -bromid und C₂₀-C₂₂-Alkyl-trimethyl-ammonium-chlorid oder -bromid; C₁₀-C₂₄-Alkyl-dimethylbenzyl-ammonium-chlorid oder -bromid, vorzugsweise C₁₂-C₁₈-Alkyl-dimethylbenzyl-ammonium-chlorid; N-(C₁₀-C₁₈-Alkyl)-pyridinium-chlorid oder -bromid, vorzugsweise N-(C₁₂-C₁₆-Alkyl)-pyridinium-chlorid oder -bromid; N-(C₁₀-C₁₈-Alkyl)-isochinolinium-chlorid, -bromid oder -monoalkylsulfat; N-(C₁₂-C₁₈-Alkylpolyoylaminoformylmethyl)-pyridinium-chlorid; N-(C₁₂-C₁₈-Alkyl)-N-methyl-morpholinium-chlorid, -bromid oder -monoalkylsulfat; N-(C₁₂-C₁₈-Alkyl)-N-ethyl-morpholinium-chlorid, -bromid oder -monoalkylsulfat; C₁₆-C₁₈-Alkl-pentaoxethyl-ammonium-chlorid; Diisobutylphenoxyethoxyethyldimethylbenzylammonium-chlorid; Salze des N,N-Diethylamino-ethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside beträgt bevorzugt 0,1 bis 10 Gew.-%, besonders bevorzugt 0,2 bis 7 Gew.-%, insbesondere besonders bevorzugt 0,5 bis 5 Gew.-% bezogen auf das fertige Mittel.

Sehr vorteilhaft sind nichtionische und amphotere Tenside.
Als nichtionische Tenside, die als waschaktive Substanzen eingesetzt werden können, kommen vorzugsweise in Betracht Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäureamidpolyethylenglykole; N-Alkyl-, N-Alkoxypolyhydroxyfettsäureamid, insbesondere Fettsäure-N-methylglucamide, Saccharoseester; Polyglykolether, Alkylpolyglycoside, Phosphorsäureester (Mono-, Di- und Triphosphorsäureester ethoxyliert und nicht-ethoxyliert).

Der Gewichtsanteil der nichtionischen Tenside in den erfindungsgemäßen Mitteln (z.B. im Falle von Rinse-off-Produkten) liegt bevorzugt im Bereich von 1 bis 20 Gew.-%, besonders bevorzugt 2 bis 10 Gew.-%, insbesondere bevorzugt 3 bis 7 Gew.-%, bezogen auf das fertige Mittel.

Bevorzugte Amphotenside sind: N-(C₁₂-C₁₈-Alkyl)-β-aminopropionate und N-(C₁₂-C₁₈-Alkyl)-β-iminodipropionate als Alkali- und Mono-, Di- und Trialkylammonium-Salze; N-Acylaminoalkyl-N,N-dimethyl-acetobetain, vorzugsweise N-(C₈-C₁₈-Acyl)aminopropyl-N,N-dimethylacetobetain; C₁₂-C₁₈-Alkyl-dimethyl-sulfopropyl-betain; Amphotenside auf Basis Imidazolin (Handelsname: Miranol^{®}, Steinapon^{®}), vorzugsweise das Natrium-Salz des 1-(β-Carboxy-methyloxyethyl)-1-(carboxymethyl)-2-lauryl-imidazoliniums; Aminoxide, z.B. C₁₂-C₁₈-Alkyldimethylaminoxid, Fettsäureamidoalkyl-dimethylaminoxid, Alkyltaurate, insbesondere Natriummethylcocoyltaurat (Hostapon^{®} CT, Clariant GmbH) Natriummethyllauroyltaurat, Isethionate, beispielsweise Natriumcocoylisethionat.

Der Gewichtsanteil der amphoteren Tenside beträgt bevorzugt 0,5 bis 20 Gew.-%, besonders bevorzugt 1 bis 10 Gew.-%, bezogen auf das fertige Mittel.

Des weiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Mitteln sind, Alkylbetaine, insbesondere Cocoamidopropylbetain, Amphoacetate, Acylglutamate, insbesondere Natriumcocoylglutamat, Alkylethersulfosuccinate, insbesondere Di-natriumlaurethsulfosuccinat, Cocosfettsäurediethanolamid, Natriumcocoylisethionat, Natriummethylcocoyltaurat und Natriummethyllauroyltaurat.

Die Gesamtmenge der in den erfindungsgemäßen Mitteln eingesetzten Tenside beträgt vorzugsweise 1 bis 70 Gew.-%, besonders bevorzugt 10 und 40 Gew.-%, insbesondere bevorzugt 12 bis 35 Gew.-%, bezogen auf das fertige Mittel.

Erfindungsgemäße, als Emulsionen vorliegende Mittel enthalten einen oder mehrere Emulgatoren. Diese Emulgatoren können ausgewählt werden aus der Gruppe der nichtionischen, anionischen, kationischen oder amphoteren Emulgatoren.

Als nichtionogene Co-Emulgatoren kommen vorzugsweise in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z.B. anionische Emulgatoren, wie mono-, di- oder tri-Phosphorsäureester, Seifen (z.B. Natriumstearat), Fettalkoholsulfate aber insbesondere kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Weiterhin können natürlich vorkommende Emulgatoren, unter denen Bienenwachs, Wollwachs, Lecithin und Sterole bevorzugt sind, verwendet werden.

Vorzugsweise sind Fettalkoholethoxylate gewählt aus der Gruppe der ethoxylierten Stearylalkohole, Cetylalkohole, Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether, Polyethylenglycol(20)cetylstearylether, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(1 6)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure oder deren Salze kann vorteilhafterweise das Natrium Laureth-11-carboxylat verwendet werden.

Als Alkylethersulfat ist Natrium Laureth-14-sulfat, als ethoxyliertes Cholesterinderivat Polyethylenglycol(30)Cholesterylether vorteilhaft. Ebenso bevorzugt ist Polyethylenglycol(25)Sojasterol.

Als ethoxylierte Triglyceride können vorteilhaft Polyethylenglykol(60)Evening Primose Glyceride verwendet werden.

Weiterhin ist es von Vorteil, die Polyethylglycolglycerinfettsäureester aus der Gruppe Polyethylenglycol(20)glyceryllaurat, Polyethylenglycol(6)glycerylcaprat/caprinat, Polyethylglycol(20)glyceryloleat, Polyethylenglycol(20)glycerylisostearat und Polyethylenglycol(18)glyceryloleat/cocoat zu wählen.

Unter den Sorbitanestern eignen sich besonders Polyethylenglykol (20)sorbitanmonolaurat, Polyethylenglycol (20)sorbitanmonostearat, Polyethylenglycol (20)sorbitanmonoisostearat, Polyethylenglycol (20)sorbitanmonopalmitat, Polyethylenglycol (20)sorbitanmonooleat.
Der Gewichtsanteil des oder der in den erfindungsgemäßen Mitteln enthaltenen Emulgators oder Emulgatoren, beträgt vorzugsweise 0,1 bis 20 Gew.-%, besonders bevorzugt 0,5 bis 15 Gew.-%, insbesondere bevorzugt 1 bis 10 Gew.-% bezogen auf das fertige Mittel.

Als kationische Polymere eignen sich vorzugsweise die unter der INCI-Bezeichnung "Polyquaternium" bekannten Verbindungen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium 37&mineral oil&PPG trideceth (^{®}Salcare SC95), PVPdimethylaminoethylmethacrylat-Copolymer, Guarhydroxypropyltriammoniumchloride, sowie Calciumalginat und Ammoniumalginat.

Des weiteren können vorzugsweise eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxypropyldiethylentriamin; Polyaminopolyamid und kationische Chitinderivate, wie beispielsweise Chitosan.

Der Gewichtsanteil an kationischen Polymeren in den erfindungsgemäßen Mitteln kann vorzugsweise im Bereich von 0,1 bis 10 Gew.-%, besonders bevorzugt im Bereich von 0,2 bis 5 Gew.-%, insbesondere bevorzugt im Bereich von 0,5 bis 2,5 Gew.-% liegen.

Die gewünschte Viskosität der Mittel kann durch Zugabe von Verdickungsmitteln eingestellt werden. In Betracht kommen vorzugsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester.

Als synthetische Polymere kommen verschiedene Materialien zum Einsatz, vorzugsweise Polyvinylalkohole, Polyacrylamide, Polyvinylamide, Polysulfonsäuren, insbesondere Copolymerisate auf Basis von Ammoniumsalzen von Acrylamido-alkylsulfonsäuren und cyclischen N-Vinylcarbonsäureamiden bzw. cyclischen und linearen N-Vinylcarbonsäureamiden oder auch hydrophob modifizierte Acrylamido-alkylsulfonsäure-Copolymerisate, Polyacrylsäure, Polyacrylsäurederivate, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den oben genannten Verbindungen, einschließlich ihrer verschiedenen Salze und Ester. Diese Polymere können wahlweise vernetzt oder unvernetzt sein.

Bevorzugte Filmbildner sind je nach Anwendungszweck Salze der Phenylbenzimidazolsulfonsäure, wasserlösliche Polyurethane, beispielsweise C₁₀-Polycarbamylpolyglycerylester, Polyvinylalkohol, Polyvinylpyrrolidoncopolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan, Vinylacetat/Crotonat, beispielsweise unter dem Handelsnamen Aristoflex^{®} A 60 (Clariant) erhältlich, sowie polymere Aminoxide, beispielsweise unter den Handelsnamen Diaformer Z-711, 712, 731, 751 erhältliche Vertreter.

Bevorzugt geeignet als antimikrobielle Wirkstoffe sind Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfa, Farnesol und Kombinationen dieser Wirksubstanzen.

Die erfindungsgemäßen Mittel enthalten die antimikrobiellen Mittel bevorzugt in Mengen bis 50 Gew.-%, besonders bevorzugt in Mengen von 0,01 bis 10 Gew.-%, insbesondere bevorzugt in Mengen von 0,1 bis 10 Gew.-%.

Vorteilhafte erfindungsgemäße Mittel enthalten ein oder mehrere Antioxidantien.
Als günstige, aber dennoch fakultativ zu verwendende Antioxidantien können alle für kosmetische und/oder pharmazeutische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. (α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbiridungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate,
α -Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haare vor oxidativer Beanspruchung schützen. Bevorzugte Antioxidantien sind dabei Vitamin E und dessen Derivate sowie Vitamin A und dessen Derivate.

Die Menge der Antioxidantien (eine oder mehrere Verbindungen) in den erfindungsgemäßen Mitteln beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Mittel.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel, zu wählen.

In einer besonders bevorzugten Ausführungsform der Erfindung enthalten die kosmetischen oder pharmazeutischen Mittel Antioxidantien ausgewählt aus Superoxid-Dismutase, Tocopherol (Vitamin E) und Ascorbinsäure (Vitamin C).

Als UV-Filter kommen vorzugsweise in Betracht 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)-benzyl]-acrylamid; 4-Methoxy-zimtsäure-2-ethyl-hexylester; ethoxyliertes Ethyl-4-aminobenzoat; 4-Methoxy-zimtsäure-isoamylester; 2,4,6-Tris-[p-(2-ethylhexyloxycarbonyl)-anilino]-1,3,5-triazin; 2-(2H-benzotriäzol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-aminocarbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Als Pigmente/Mikropigmente können vorzugsweise mikrofeines Titandioxid, Glimmer-Titanoxid, Eisenoxide, Glimmer-Eisenoxid, Zinkoxid, Siliziumoxide, Ultramarinblau, Chromoxide, eingesetzt werden.

Als Gelierungsmittel eignen sich alle oberflächenaktiven Stoffe, die in der flüssigen Phase gelöst eine Netzwerkstruktur ausbilden und so die flüssige Phase verfestigen.
Geeignete Geliermittel sind z.B. in der WO 98/58625 genannt.

Bevorzugte Gelierungsmittel sind Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher.

Bevorzugt enthalten die erfindungsgemäßen Mittel 0,01 bis 20 Gew.-%, besonders bevorzugt 0,1 bis 10 Gew.-%, insbesondere bevorzugt 1 bis 8 Gew.-% und ganz besonders bevorzugt 3 bis 7 Gew.-% an Geliermitteln.

Weitere Zusatzstoffe können Siliconverbindungen sein, vorzugsweise Dimethylpolysiloxane, Methyl-phenylpolysiloxane, cyclische Silicone sowie amino-, fettsäure-, alkohol-, polyether-, epoyx-, fluor- und/oder alkylmodifizierte Siliconverbindungen, beispielsweise Phenyltrimethicone der Clariant GmbH wie SilCare^{®} 15M30, SilCare^{®} 15M40, SilCare^{®} 15M50, SilCare^{®} 15M60, Caprilyltrimethicone wie SilCare^{®} 31M30, SilCare^{®} 31 M40, SilCare^{®} 31 M 50, SilCare^{®} 31 M 60, Alkylmethicone wie SilCare^{®} Silicone 41M10, SilCare^{®} Silicone 41 M15, SilCare^{®} Silicone 41 M20, SilCare^{®} Silicone 41 M30, SilCare^{®} 41 M40, SilCare^{®} 41 M50, SilCare^{®} 41 M65, SilCare^{®} 41 M70 oder SilCare^{®} 41 M80, SilCare^{®} 41 M90,Trimethylsilyltrimethylsiloxylactat, Trimethylsilyltrimethylsiloxyglycolat, Trimethylsilyltrimethylsiloxysalicylat, Retinoxytrimethylsilan, Polyalkylarylsiloxane und Polyethersiloxan-Copolymere und modifizierte Polyorganosiloxane beispielsweise SilCare^{®} Silicone SEA (Clariant GmbH).

Die erfindungsgemäßen Mittel können die oben genannten Siliconverbindungen vorzugsweise in den Gewichtsmengen von 0,1 bis 20 Gew.-%, besonders bevorzugt 0,2 bis 15 Gew.-%, insbesondere bevorzugt 0,5 bis 10 Gew.-%, bezogen auf die fertigen Mittel, enthalten.

Als Trägermaterialien in Betracht kommen vorzugsweise pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und CelluloseDerivate.

Als fungizide Wirkstoffe können vorzugsweise Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Octopirox in den Gewichtsmengen von 0,05 bis 5 Gew.-%, bevorzugt 0,1 bis 3 Gew.-%, besonders bevorzugt 0,2 bis 2 Gew.-%, bezogen auf die fertigen Mittel eingesetzt werden. Die erfindungsgemäßen Mittel können vorteilhaft mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen, abgemischt werden.

Als feuchtigkeitsspendende Substanz stehen vorzugsweise Isopropylpalmitat, Glycerin und/oder Sorbitol zu Verfügung, die bevorzugt in den Gewichtsmengen 0,1 bis 50 % eingesetzt werden.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide verwendet werden.

Als Konservierungsmittel in Betracht kommen vorzugsweise Phenoxyethanol, Parabene, Pentandiol oder Sorbinsäure. Sie werden vorzugsweise in den Gewichtsmengen von 0,001 bis 5 Gew.-%, besonders bevorzugt von 0,01 bis 3 Gew.-%, insbesondere bevorzugt von 0,1 bis 2 Gew.-%, bezogen auf die fertigen Mittel eingesetzt.

Als Farbstoffe können die für kosmetische und pharmazeutische Zwecke geeigneten und zugelassenen Substanzen verwendet werden.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z.B. die lonone, alpha-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Säuren oder Laugen zur pH-Wert Einstellung werden vorzugsweise Mineralsäuren, beispielsweise HCl, anorganische Basen, beispielsweise NaOH, KOH und organische Säuren, bevorzugt Zitronensäure verwendet.

Die Mittel sind vorzugsweise auf einen pH Wert im Bereich 2 bis 10, bevorzugt pH 3 bis 8, besonders bevorzugt 4 bis 7 eingestellt.

Die nachfolgenden Beispiele sollen die Erfindung in nicht einschränkender Weise näher erläutern. Die Angabe "Teile" ist als "Gewichtsteile" zu verstehen.

### Beispiele

### A. Silikonöle

### 1. Herstellung der Silangemische (I) und (II)

### 1.1 Herstellung von Glycidyldiethylamin

298,00 Teile Diethylamin wurden mit 12,25 Teilen Wasser vermengt. Danach wurden unter Rühren, bei 20 °C, innerhalb von 10 Stunden 377,60 Teile Epichlorhydrin zugetropft. Anschließend wurde noch während weiteren 10 Stunden bei 20°C weitergerührt und es wurden danach 506,7 Teile Natriumhydroxyd-Lösung, wässrig, 30 gew.-%ig, zugetropft. Nach 3 Stunden (15 - 20 °C) wurde der Rührer abgestellt. Es bildete sich eine organische Phase (501,5 Teile) die abgetrennt wurde. Sie bestand aus ca. 384,0 Teilen Glycidyldiethylamin, 60,0 Teilen N,N,N',N'-Tetraethyl-1,3-diamino-2-hydroxy-propan, 25,0 Teilen Wasser, 24,5 Teilen N,N-Diethyl-2-hydroxy-3-chloro-propanamin, 1,0 Teil Natriumchlorid und 7,0 Teilen 3-Dimethylamino-2-hydroxy-1-propanol.

### 1.2 Herstellung des Silangemisches (I)

309,00 Teile 3-(2-Aminoethylamino)-propyl-dimethoxymethylsilan wurden mit 505,40 Teilen der frisch hergestellten organische Phase von 1.1 unter Rühren vermischt und auf 60°C aufgeheizt. Es fand eine leicht exotherme Reaktion statt. Nach ca. 2 Stunden klang die exotherme Reaktion ab und es wurde während 4 Stunden bei 60°C weiter reagieren lassen. Danach wurde auf Raumtemperatur abgekühlt. Es konnten keine Glycidylgruppen mehr titriert werden. Es hatte nämlich eine Alkylierung der primären Aminogruppe stattgefunden. Es wurden so 814,4 Teile eines Silangemisches (I) erhalten mit folgenden Hauptkomponenten:

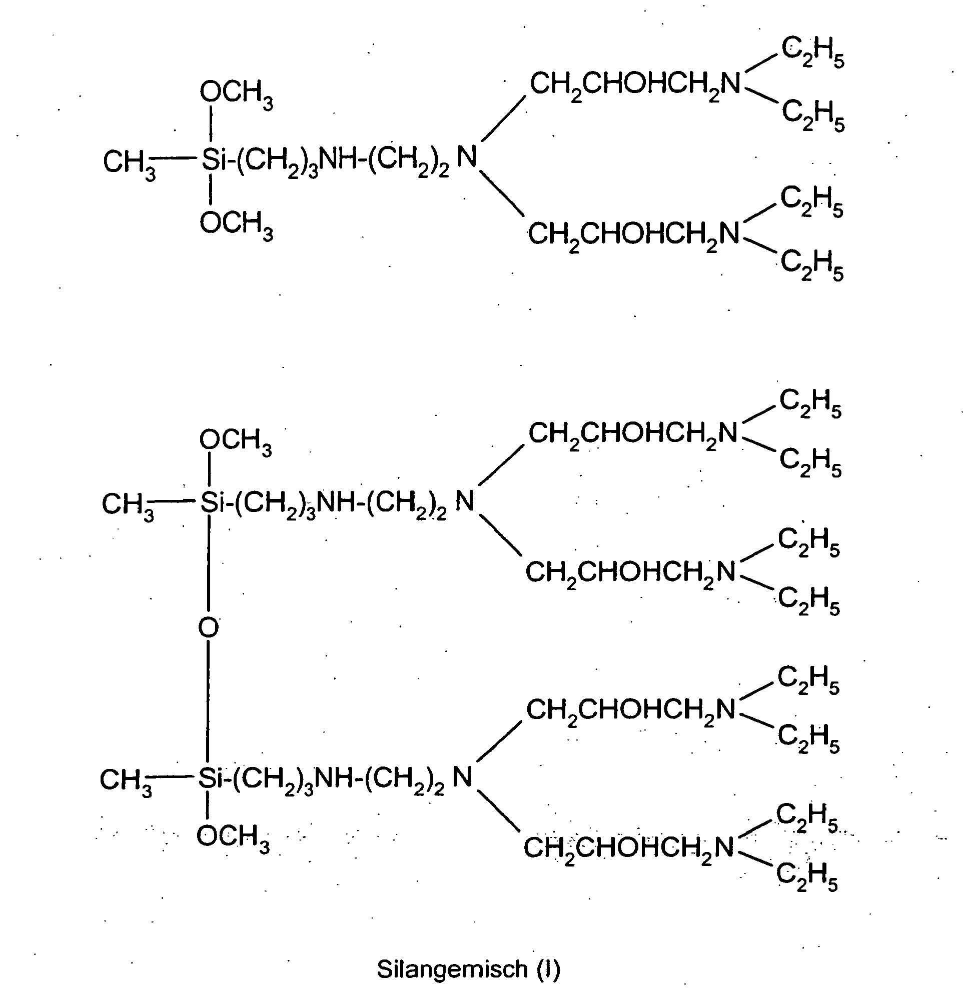

### 1.3 Herstellung des Silangemisches (II)

286,50 Teile 3-Aminopropyl-diethoxy-methylsilan wurden mit 505,40 Teilen der frisch hergestellten organischen Phase von 1.1 unter Rühren bei Raumtemperatur zusammengemischt und auf 60°C aufgeheizt. Es fand eine exotherme Reaktion statt wobei die Temperatur durch Kühlen bei 60°C gehalten wurde. Sobald die exotherme Reaktion vorbei war wurde noch über 4 Stunden bei 60°C weiter reagieren gelassen und dann erst auf Raumtemperatur abgekühlt. Es konnten keine Glycidylgruppen mehr titriert werden. Es hatte nämlich eine Alkylierung der primären Aminogruppen des Silans stattgefunden. Man erhielt so 791,9 Teile eines Silangemisches (II) mit folgenden Hauptkomponenten:

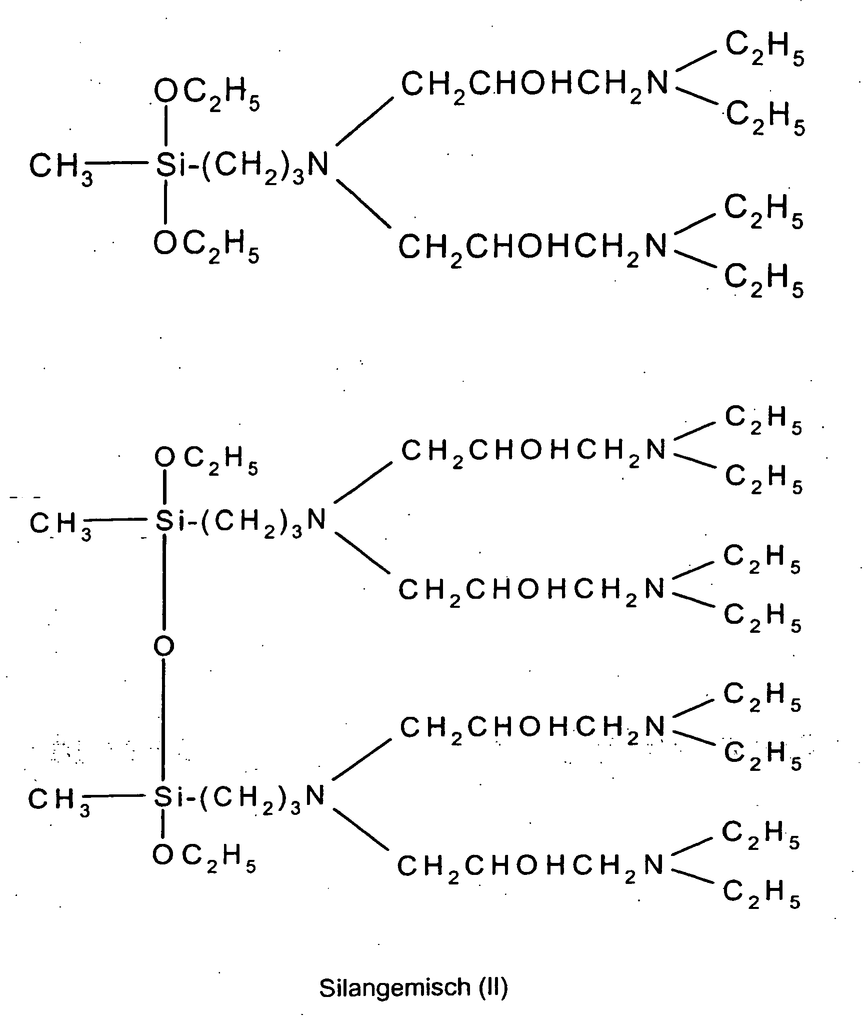

### 2. Herstellung der Silane (III) und (IV)

### 2.1 Herstellung von Glycidyldipropylamin

404,0 Teile Dipropylamin wurden mit 12,0 Teilen Wasser vermengt und auf eine Temperatur von 20°C gekühlt. Dann wurden innerhalb von 60 Minuten 370,0 Teile Epichlorhydrin zugetropft, wobei die Temperatur zwischen 18 und 20°C gehalten wurde. Nach einer Nachrührzeit von ca. 20 Stunden bei 20°C wurden dann 673,4 Teile wässrige Natriummethylat-Methanol-Lösung, 30-%ig, in 60 Minuten zugetropft. Es bildete sich sofort ein Natriumchlorid-Niederschlag durch die Bildung der Glycidyl-Verbindung. Nach der Entfernung des Kochsalzes durch Filtration wurde zuerst Methanol und danach das gebildete Glycidyldipropylamin abdestilliert. Es wurden zwischen 65 und 80°C bei 8 bis 14 mbar 470 g Glycidyldipropylamin mit einem Äquivalentgewicht von 161,8 (97 %-ig) erhalten (Ausbeute: 72,5 %).

### 2.2 Herstellung des Silans (III)

309,00 Teile 3-(2-Aminoethylamino)propyl-dimethoxymethylsilan wurden genau wie unter 1.2 beschrieben mit 485,4 Teilen Glycidyldipropylamin hergestellt und gemäss 2.1 umgesetzt. So wurden 794,4 Teile Silan (III) folgender Struktur erhalten:

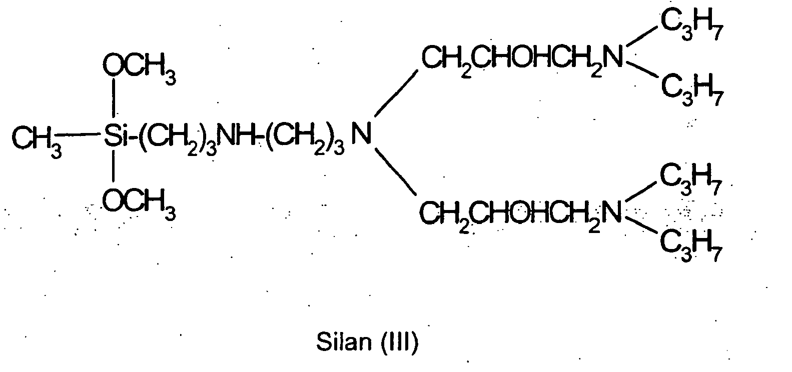

### 2.3 Herstellung des Silans (IV)

286,50 Teile 3-Aminopropyl-diethoxy-methylsilan wurden genau wie unter 1.2 beschrieben mit 485,4 Teilen Glycidyldipropylamin hergestellt und gemäss 2.1 umgesetzt. So wurden 771,9 Teile Silan (IV) folgender Struktur erhalten:

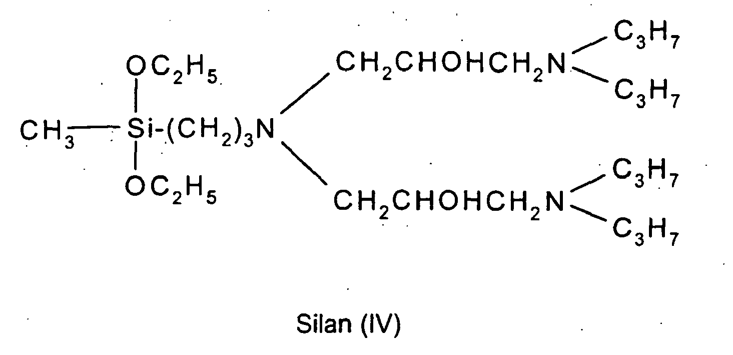

### 3. Herstellung des Silans (V)

187,0 Teile N'-[3-(Dimethylamino)propyl]-N,N-dimethylpropan-1,3-diamin wurden auf 80°C aufgeheizt. Dann wurden 248,0 Teile Diethoxy-(3-glycidyloxypropyl)-methylsilan zugetropft, wobei die Temperatur bei 80°C gehalten wurde. Nach der Glycidylzugabe wurde noch über weitere 4 Stunden bei 130°C ausreagieren gelassen. So wurden 435,0 Teile Silan (V) folgender Struktur erhalten:

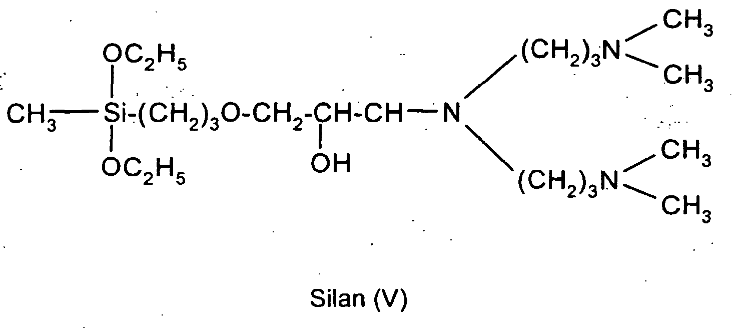

### 4. Herstellung des Polysiloxans (I)

691,0 Teile Polydimethylsiloxandiol (Viskosität 80 cp = 0,08 Pa·s) (Polydimethylsiloxandiol L), 28,2 Teile Silangemisch (I) sowie 5,5 Teile einer 40 %igen Lösung von Benzyltrimethylammoniumhydroxyd in Methanol wurden zusammengemischt und unter Rühren auf eine Temperatur von 80°C erhitzt. Nach 3 Stunden bei 80°C wurde bis auf ca. 200 mbar Restdruck evakuiert und bei diesem Druck innerhalb von 60 Minuten auf 150°C aufgeheizt. Dann wurde auf ca. 50 mbar Restdruck evakuiert, und nach 60 Minuten unter diesen Bedingungen unter konstantem Restdruck (50 mbar) auf Raumtemperatur abgekühlt. So wurden ca. 707,0 Teile Polysiloxan (I) (Viskosität 2660 cp = 2,66 Pa·s) und 15,0 Teile Destillat erhalten.

### 5. Herstellung des Polysiloxans (II)

691,0 Teile Polydimethylsiloxandiol (Viskosität 80 cp = 0,08 Pa·s) (Polydimethylsiloxandiol L), 38,73 Teile Silangemisches (II) sowie 5,4 Teile einer 40-%igen Lösung von Benzyltrimethylammoniumhydroxyd in Methanol wurden unter Rühren auf 80°C erhitzt. Nach 3 Stunden bei 80°C wurde auf ca. 200 mbar Restdruck evakuiert und bei diesem Restdruck auf 150°C aufgeheizt (in ca. 60 Minuten). Danach wurde auf 50 mbar Restdruck evakuiert und es wird während 60 Minuten bei diesem Druck und bei 150°C destilliert. So wurden ca. 15,8 Teile Destillat erhalten. Nach dem Abkühlen auf Raumtemperatur (unter Vakuum) wurden ca. 715,4 Teile Polysiloxan (II) (Viskosität 900 cp = 0,9 Pa.s) erhalten.

### 6. Herstellung des Polysiloxans (III)

691,0 Teile Polydimethylsiloxandiol, 55,1 Teile Silan (III) und 3,2 Teile einer 40 %igen Lösung von Benzyltrimethylammoniumhydroxyd in Methanol wurden in einem geschlossenen Gefäss auf 80°C erhitzt. Nach 4 Stunden bei 80°C wurde der Druckreaktor mit einer Destillationsbrücke versehen und auf 200 mbar Restdruck evakuiert. Sobald dieser Druck erreicht war, wurde innerhalb von 60 Minuten auf 150°C aufgeheizt. Anschließend wurde der Restdruck auf 50 mbar erniedrigt und während 1 Stunde bei 150°C weitergerührt. Danach wurde bei 50 mbar Restdruck auf Raumtemperatur abgekühlt. So wurden ca. 728,0 Teile Polysiloxan (III) mit einer Viskosität von 2150 cp = 2,15 Pa·s erhalten.

### 7. Herstellung des Polysiloxans (IV)

691,0 Teile Polydimethylsiloxandiol L, 38,0 Teile Silan (IV) und 0,7 Teile einer 40 %igen Lösung von Benzyltrimethylammoniumhydroxyd in Methanol wurden auf 80°C erhitzt und über eine Zeitdauer von 3 Stunden bei dieser Temperatur reagieren gelassen. Danach wurde auf 900 mbar Restdruck evakuiert und bei diesem Druck innerhalb von 60 Minuten auf 150 °C aufgeheizt. Dann wurde evakuiert bis 50 mbar Restdruck erreicht waren und während 30 Minuten die Temperatur bei 150°C gehalten. Anschließend wurde auf Raumtemperatur abgekühlt und mit Stickstoff auf Normaldruck gebracht. Es wurden 694,1 Teile Polysiloxan (IV) mit einer Viskosität von 1760 cp = 1,76 Pa·s erhalten.

### 8. Herstellung des Polysiloxans (V)

Es wurde vorgegangen wie für das Polysiloxan (IV), aber es wurden 32,1 Teile Silan (V) an Stelle von 38,0 Teilen Silan (IV) eingesetzt. So wurden 696,2 Teile Polysiloxan (V) mit einer Viskosität von 1200 cp = 1,2 Pa.s erhalten.

### 9. Herstellung des Polysiloxans (VI)

419,3 Teile Octamethylcyclotetrasiloxan (D4) und 25,3 Teile 1,1,3,3-Tetramethyldisiloxan wurden zusammen mit 0,43 Teilen Trifluoromethan-sulfonsäure auf 80°C aufgeheizt. Nach 4 Stunden bei 80°C wurden 0,43 Teile Magnesiumoxid zugegeben, auf 50 mbar Restdruck evakuiert und unter diesen Bedingungen auf 150°C aufgeheizt. Nach 30 Minuten bei 150°C und 50 mbar wurde unter Vakuum auf Raumtemperatur abgekühlt und über einen Papierfilter ausgeladen. So wurden 405,7 Teile eines H-terminierten Polydimethyjsiloxans erhalten. Dieses Produkt wurde dann unter Stickstoff wieder auf 80°C erhitzt. Sobald diese Temperatur erreicht war, wurden 35 ml einer 3-%igen (auf Platin bezogen) eines Platin-Cyclovinylmethylsiloxan-Komplexes (in cyclischen Methylvinylsiloxanen) (Hydrosilylierungskatalysator) zugefügt sowie 42,6 Teile Allylglycidylether innerhalb von ca. 60 Minuten zugetropft. Sobald die Si-H-Gruppen abreagiert hatten (wenn nicht, muss noch etwas Katalysator zugegeben werden) wurde auf 100°C aufgeheizt, auf 50 mbar Restdruck evakuiert und 60 Minuten lang bei 100°C gehalten. Dann wurde auf Raumtemperatur abgekühlt. So wurden 443,0 Teile Glycidyl-terminiertes Polydimethylsiloxan mit einem Äquivalentgewicht von 1334 (Äquivalentgewicht für eine Glycidylgruppe) erhalten. Nun wurden 62,1 Teile N,N,N',N'-Tetramethyl-dipropylen-triamin zugefügt und auf 130°C aufgeheizt. Sobald die Glycidylgruppen nicht mehr titrierbar waren, wurde auf Raumtemperatur abgekühlt. So wurden 505,1 Teile Polysiloxan (VI) erhalten mit folgender allgemeinen Formel:

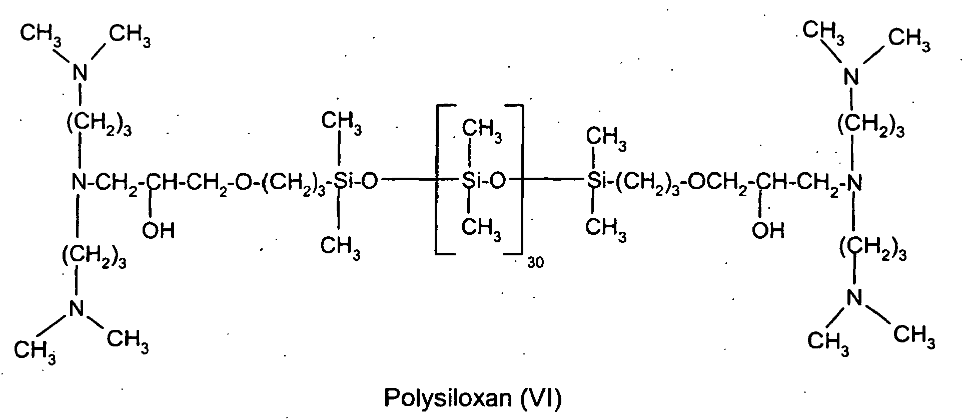

### B. Endprodukte

### 1. Herstellung von quaternärem Polysiloxan (I)

200,0 Teile Polysiloxan (I) wurden mit 50,0 Teilen Tridecanolpoly-6,5-ethylenglykol (Emulgator I) und 50,0 Teilen Wasser emulgiert und auf 40°C aufgeheizt. Sobald diese Temperatur erreicht war, wurden 10,04 Teile Dimethylsulfat zugetropft. Nach 6 Stunden bei 40°C wurden vorerst zweimal 200 Teile Wasser und anschließend 40 Teile Hexylenglykol zugefügt. Danach wurden noch 70 Teile Emulgator (I) und 180 Teile Wasser zugegeben. So werden 1000,0 Teile einer 20 %igen Mikroemulsion des voll quaternären Polysiloxans (I) erhalten (quat. Polysiloxan I). 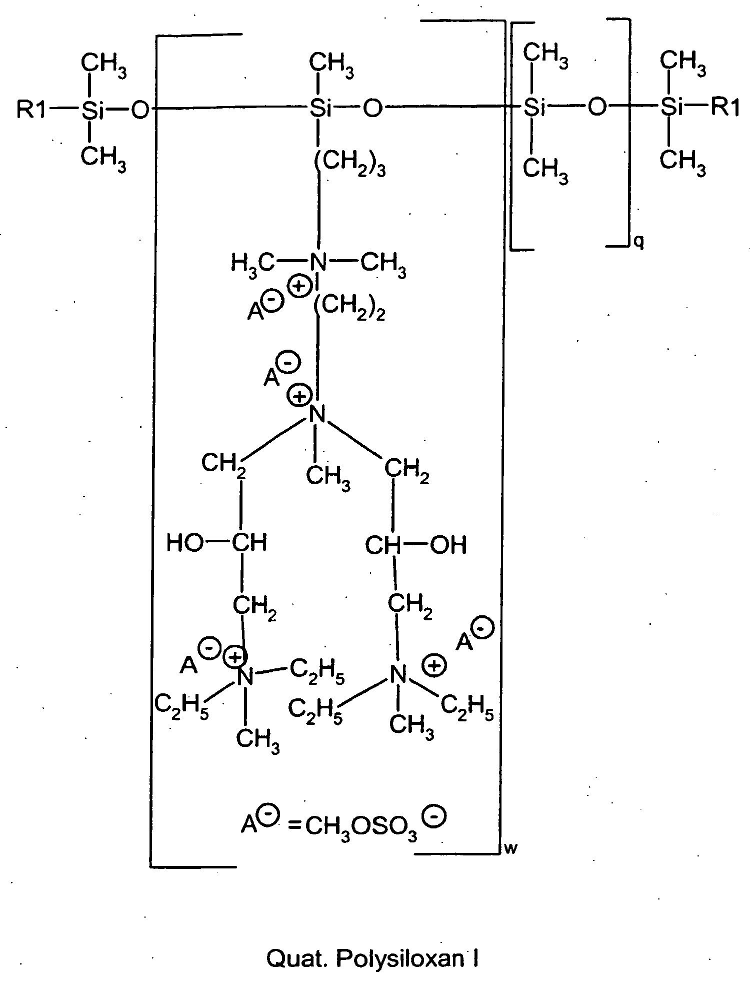 R1 = OH, OMe
w:q~1:40

### 2. Herstellung von quaternärem Polysiloxans (la)

Es wurde vorgegangen wie zur Herstellung von quat. Polysiloxan I, aber an Stelle von 10,04 Teilen Dimethylsulfat wurden nur 6,02 Teile zugefügt und vor dem Aufheizen auf 40°C wurden nach der Zugabe von 50 Teilen Emulgator (I) 52,0 Teile Wasser an Stelle von 50 Teilen verwendet und noch 2,0 Teile Dimethyldicarbonat zugefügt. Sobald die CO₂-Entwicklung stattgefunden hatte wurde auf 40°C aufgeheizt und weitergefahren. So wurden 1000,0 Teile einer 20 %-igen Mikroemulsion eines quat. Polysiloxans la erhalten mit folgenden funktionellen Gruppen:

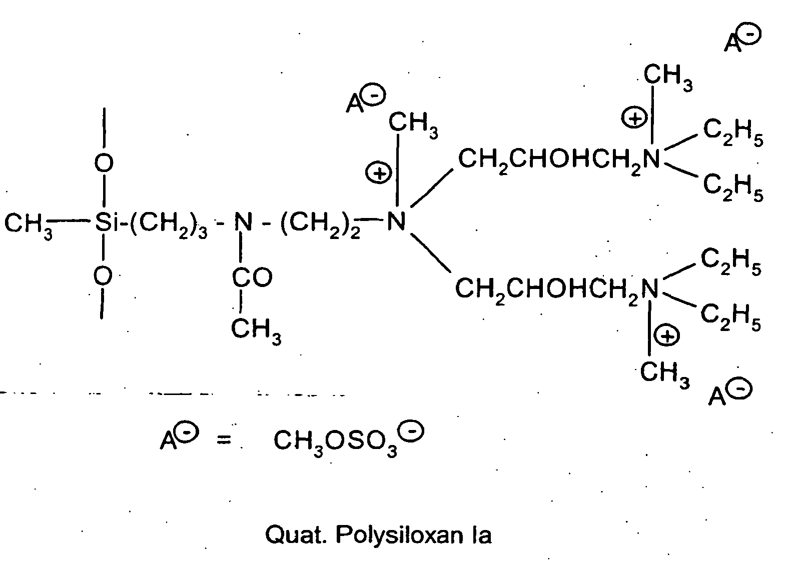

### 3. Herstellung von quaternärem Polysiloxan (II)

200,0 Teile Polysiloxan (II) wurden mit 40,0 Teilen Hexylenglykol vermischt und auf 40°C aufgeheizt. Dann wurden 8,86 Teile Dimethylsulfat zugetropft und während 6 Stunden bei 40°C ausreagieren gelassen. Danach wurden 115,0 Teile Emulgator (I) und - sobald eine homogene Mischung vorlag - 390,0 Teile Wasser von 60°C zugefügt. Es bildete sich eine Mikroemulsion, die durch Zugabe von 247,0 Teilen Wasser und durch externe Kühlung auf Raumtemperatur abgekühlt wurde. So wurden ca. 1000 Teile Mikroemulsion (quat. Polysiloxan II) erhalten. Das quat. Polysiloxan II enthält folgende funktionelle Gruppen:

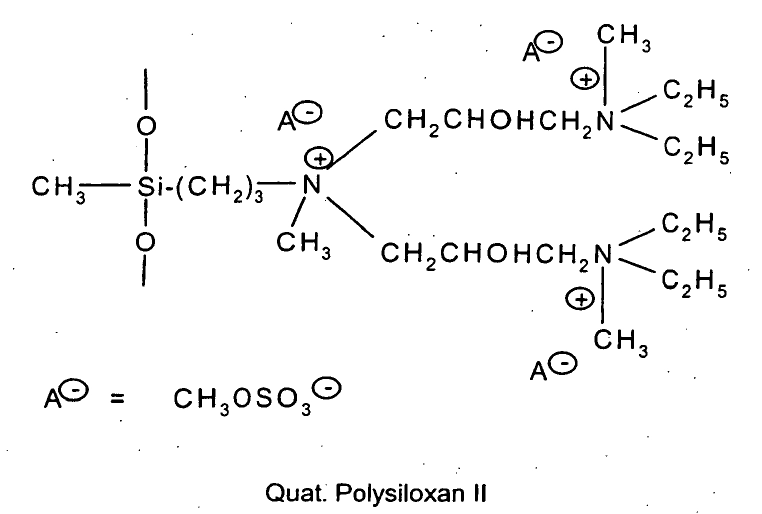

### 4. Herstellung von quaternärem Polysiloxan (III)

Es wurde vorgegangen wie für quat Polysiloxan II aber mit folgenden Mengen und Edukten:

| | |
|---|---|
| Polysiloxan (III) | 200,0 Teile |
| Wasser (1) | 52,0 Teile |
| Emulgator (I) (1) | 50,0 Teile |
| Dimethyldicarbonat | 3,8 Teile |
| Dimethylsulfat | 10,8 Teile |
| Wasser (2) | 400,0 Teile |
| Hexylenglykol | 40 Teile |
| Emulgator (I) (2) | 70 Teile |
| Wasser (3) | 174 Teile |

So wurden ca. 1000,0 Teile Quat Polysiloxan III erhalten. Das emulgierte quat Polysiloxan III wies folgende funktionellen Gruppen auf:

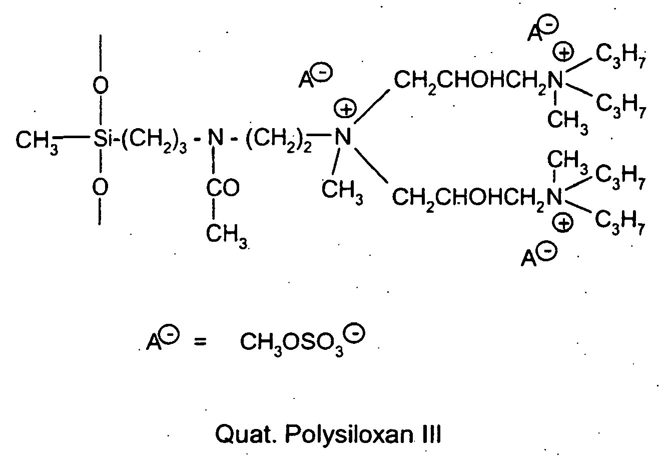

### 5. Herstellung von quaternärem Polysiloxan (IV)

Es wurde vorgegangen wie für Quat. Polysiloxan III aber mit folgenden Mengen und Edukten:

| | |
|---|---|
| Polysiloxan (IV) | 200,0 Teile |
| Hexylenglykol | 40,0 Teile |
| Dimethylsulfat | 8,1 Teile |
| Emulgator (I) | 115,0 Teile |
| Wasser (1) | 390,0 Teile |
| Wasser (2) | 247,0 Teile |

So wurden ca. 1000,0 Teile quat. Polysiloxan IV erhalten. Das emulgierte quat. Polysiloxan IV wies folgende funktionellen Gruppen auf:

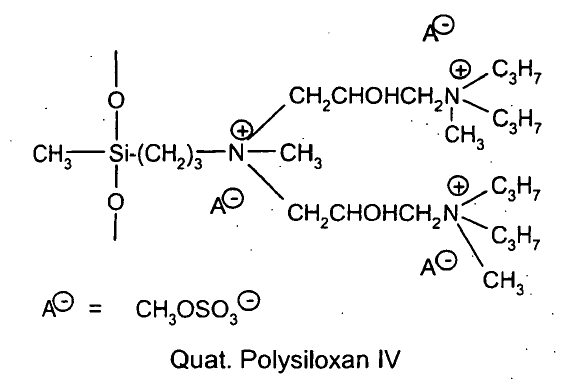

### 6. Herstellung von quaternärem Polysiloxan (V)

Es wurde vorgegangen wie für quat. Polysiloxan IV, aber mit dem Polysiloxan (V) an Stelle von (IV). Es wurden ca. 1000,0 g Quat. Polysiloxan V erhalten. Das emulgierte Quat. Polysiloxan V wies folgende funktionellen Gruppen auf:

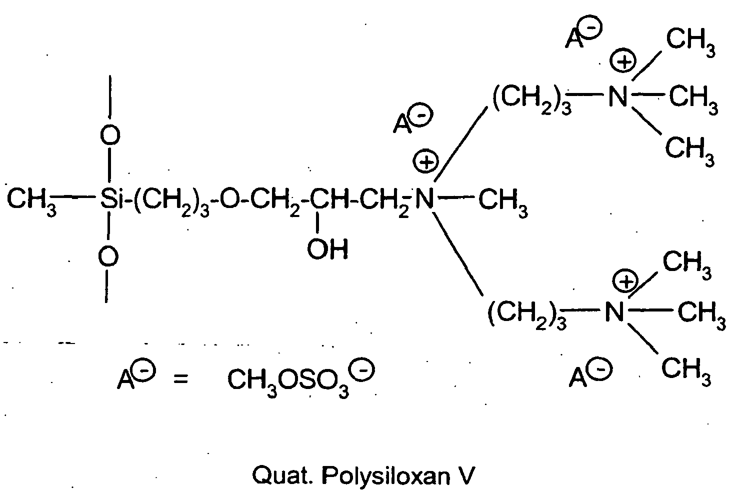

### 7. Herstellung von quaternärem Polysiloxan (VI)

200,0 Teile Polysiloxan (VI) wurden mit 100,0 Teilen Hexylenglykol vermischt und bei 40°C mit 49,7 Teilen Dimethylsulfat während 4 Stunden zur Reaktion gebracht. Nach der Zugabe von 751 Teilen Wasser wurden 1000,0 Teile Endprodukt quat. Polysiloxan VI erhalten. Das selbstdispergierte quat. Polysiloxan VI wies folgende Struktur auf:

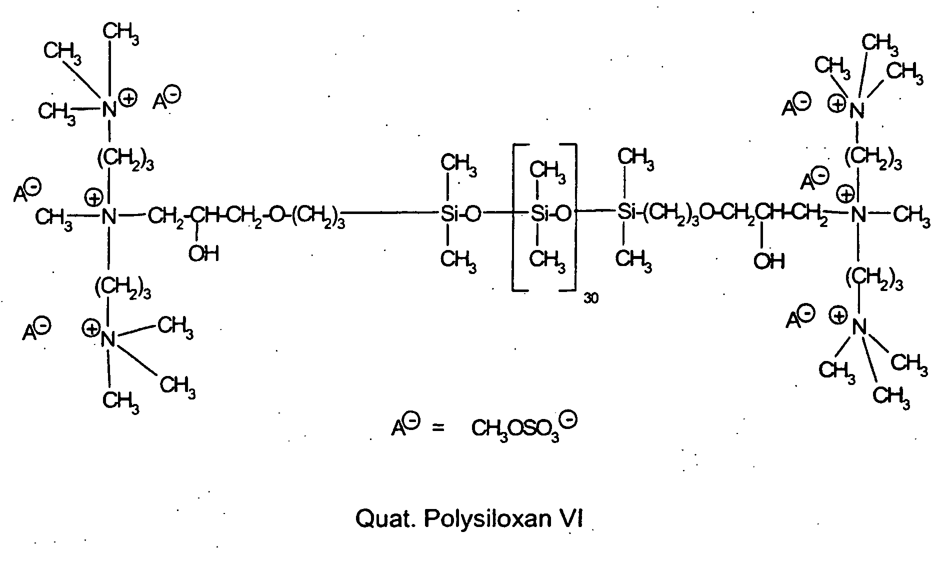

### Beispiel 1, Farbschutz und Glanz

Standardisierte, blond gebleichte Haarsträhnen wurden mit einer handelsüblichen Permanenthaarfarbe (Viva Purered, Glutrot) unter Standardbedingungen gefärbt. Anschließend wurde die Strähne A mit Laurylethersulfat : Cocoamidopropylbetain (3:1, 12 Gew.-% Aktivgehalt, H₂O ad 100 Gew.-%) und die Strähne B mit Laurylethersulfat : Cocoamidopropylbetain (3:1, 12 Gew.-% Aktivgehalt, H₂O ad 100 % Al) + quat Polysiloxan I(1 Gew.-% Aktivgehalt) je 4x gewaschen. In einem 10 Personen umfassenden Panel wurd die Strähne A als Standard (0) gesetzt. Eine Verbesserung gegenüber dem Standard wurde mit + (= gut), ++ (= sehr gut) oder +++ (=-herausragend) bewertet, eine Verschlechterung mit - oder - - benotet.

**Tabelle 1: Anwendungsbeispiel Farbschutz und Glanz**

| Nachfolgend sind die Parameter Farbintensität, Farbbrillance, Glanz, Griff und elektrostatische Aufladung nach den vier Waschvorgängen aufgeführt (Durchschnitt aus allen Testpersonen). | | | | | |
|---|---|---|---|---|---|
| | Farbintensität | Farbbrillanz | Glanz | Griff | elektrostatische Aufladung |
| Strähne A (Standard) | 0 | 0 | 0 | 0 | 0 |
| Strähne B | + | ++ | +++ | + | 0 |

### Ergebnis:

Die mit quat. Polysiloxan I behandelten Haarsträhnen wiesen nach optischer und sensorischer Prüfung ein deutliches geringeres Ausbluten der Haarfarbe auf (erhöhte Farbintensität, höhere Farbbrillanz) und zeigten zusätzlich einen signifikant verbesserten Glanz und einen besseren Griff.

### Beispiel 2: Kämmbarkeit von Haaren

Blonde, gebleichte Haarsträhnen wurden mit einer 2 %-igen (aktiv) wässrigen Lösung von quat. Polysiloxan I behandelt und bzgl. Naß- und Trockenkämmbarkeit durch ein 10 Personen umfassendes Testpanel geprüft (Strähne B). Genamin CTAC (INCl: Cetrimonium Chloride) dient dabei als Standard (Strähne A).

Die Bewertung erfolgt von -2 (viel schlechter) über 0 (ähnlich wie Standard) bis zu +2 (sehr viel besser) in Einerschritten.

Die Ergebnisse der Tabelle 2 stellen Mittelwerte dar.

**Tabelle 2: Kämmbarkeit von Haaren**

| | Nass | Trocken |
|---|---|---|
| Strähne A (Standard) | 0 | 0 |
| Strähne B | +1,5 | -1,5 |

Die Ergebnisse zeigen, dass quat. Polysiloxan I die Nasskämmbarkeit gegenüber dem Standard signifikant erhöht. Die etwas schlechteren Werte bei der Trockenkämmbarkeit lassen sich durch Zugabe anderer Additive, wie z.B. SilCare Silicone SEA, ausgleichen.

Die nachfolgenden Anwendungen sollen die Erfindung näher erläutern, ohne sie jedoch darauf zu beschränken (bei allen Prozentangaben handelt es sich um Gewichtsprozent).

### Beispiel 3: Creme-Spülung

| | | | |
|---|---|---|---|
| A | Hostacerin^{®} DGI | Clariant | 1,50 % |
| | Cetylalkohol | | 3,00 % |
| B | Genamin^{®} CTAC | Clariant | 3,30 % |
| | Wasser | | ad 100 % |
| | Konservierungsmittel | | q.s. |
| C | Duftstoff | | 0,30 % |
| | quat. Polysiloxan I | Clariant | 1,25 |

### Herstellung:

- I: Schmelzen von A bei ca. 75°C
- II: Erwärmen von B auf ca. 75°C
- III: Zugabe von II zu I unter Rühren und weiterrühren bis 30°C
- IV: Zugabe von C zu III bei 30°C
- V: Einstellen auf pH 4,0 mit Zitronensäure

### Beispiel 4: Creme-Spülung

| | | | |
|---|---|---|---|
| A | Hostacerin^{®} DGI | Clariant | 1,50% |
| | Cetylalkohol | | 4,00 % |
| B | Genamin^{®} CTAC | Clariant | 3,30 % |
| | Wasser | | ad 100 % |
| | Konservierungsmittel | | q.s. |
| C | Duftstoff | | 0,30 % |
| | quat. Polysiloxan I | Clariant | 0,75 |
| | SilCare^{®} Silicone SEA | (Clariant) | 0,75 |

### Herstellung:

- I: Schmelzen von A bei ca. 75°C
- II: Erwärmen von B auf ca. 75°C
- III: Zugabe von II zu I unter Rühren und weiterrühren bis 30°C
- IV: Zugabe von C zu III bei 30°C
- V: Einstellen auf pH 4,0 mit Zitronensäure

### Beispiel 5: Creme-Spülung mit Perlglanzeffekt

| | | | |
|---|---|---|---|
| A | Hostacerin^{®} DGI | Clariant | 1,50 % |
| | Cetylalkohol | | 4,00 % |
| B | Genamin^{®} BTLF | Clariant | 3,30% |
| | Wasser | | ad 100 % |
| | Konservierungsmittel | | q.s. |
| C | Duftstoff | | 0,30 % |
| | quat. Polysiloxan I | Clariant | 1,25 |
| D | Zitronensäure | | q.s. |

### Herstellung:

- I: Schmelzen von A bei ca. 75°C
- II: Erwärmen von B auf ca. 75°C
- III: Zugabe von II zu I unter Rühren und weiterrühren bis 30°C
- IV: Zugabe von C zu III bei 30°C
- V: Einstellen auf pH 4,0 mit Zitronensäure

### Beispiele 6 bis 13: Haarschampoo

| | Inhaltsstoffe | 6 | 7 | 8 | 9 | 10 | 11 | 12 | 13 |
|---|---|---|---|---|---|---|---|---|---|
| A | Genapol LRO liq | 39 | 39 | 39 | 39 | 39 | 39 | 39 | 39 |
| | Genagen CAB 818 | 15 | 15 | 15 | 15 | 15 | 15 | 15 | 15 |
| | aqua dest. | ad 100 | ad 100 | ad 100 | ad 100 | ad 100 | ad 100 | ad 100 | ad 100 |
| B | Genapol L3 | 1 | 1 | 1,25 | 1 | 1 | 1 | 1 | 1 |
| | quat. Polysiloxan I | 1,25 | 1,25 | 1,25 | 1,25 | 1,25 | 1,25 | 1,25 | 1,25 |
| | NaCl | 2,5 | 2,5 | 2,5 | 2,5 | 2,5 | 2,5 | 2,5 | 2,5 |
| | hydrolized silk | | 1 | | | | | | |
| | Guar | | | 1 | | | | | |
| | Octopirox | | | | 0,3 | | | | |
| | SilCare SiliconSEA | | | | | 0,5 | | | |
| | Genapol PGL | | | | | | 1 | | 1 |
| | Eusolex 232 | | | | | | | | 0,1 |
| | Uvinol P25 | | | | | 0,1 | | | |
| | Uvinol MS 40 | | | 0,1 | | | | | |
| | Farbstoffe, Duftstoffe | q.s | q.s | q.s | q.s | q.s | q.s | q.s | q.s. |
| | Zitronensäure oder NaOH | q.s. | q.s. | q.s. | q.s. | q.s. | q.s. | q.s. | q.s. |
| C | NaCl | 2,5 | 2,5 | 2,5 | 2,5 | 2,5 | 2,5 | 2,5 | 2,5 |

### Herstellung:

- I: Mischen der Komponenten A
- II: Nacheinander Zugabe der Komponenten B zu I
- III: Einstellen des pH-Wertes mit Zitronensäure oder NaOH
- IV: Einstellen der Viskosität mit NaCl

### Beispiel 14: Shampoo mit Farbschutz für gefärbtes Haar

| | | | |
|---|---|---|---|
| A | Glucamat DOE-120 | Clariant | 2,00 % |
| | Emulsogen^{®} HCO 040 | | 2,00 % |
| B | Wasser | | ad 100 % |
| C | Genapol^{®} LRO flüssig | Clariant | 22,22 % |
| | Genagen^{®} KB | Clariant | 13,33% |
| | Genamin^{®} KSL | Clariant | 3,33% |
| | Aristoflex^{®} PEA 70 | Clariant | 2,86 % |
| | Sandopan^{®} DTC, Säure | Clariant | 2,20 % |
| | NIGAGUARD^{®} DCB | Clariant | 0,10 % |
| | quat. Polysiloxan I | Clariant | 0,50 % |
| | Farbstoff | | q.s. |
| | Duftstoff | | 0,20 % |
| D | NaOH | | |

### Herstellung:

- I: Einrühren der Komponenten A in B und Erwärmen auf ca. 60°C und unter Rühren, Abkühlen auf Raumtemperatur
- II: Nacheinander Einrühren der Komponenten C in I
- III: Rühren bis Formulierung klar erscheint
- IV: Einstellen auf pH 5,5 mit D

### Beispiel 15: Tönungsshampoo

| | | | |
|---|---|---|---|
| A | Genagen^{®} KB | Clariant | 7,00 % |
| | Velsol Semipermanentfarbstoff | Clariant | 0,50 % |
| B | Genapol^{®} T 500 P | Clariant | 0,50 % |
| | Wasser | | ad 100 % |
| C | Genapol^{®} LRO flüssig | Clariant | 30,00 % |
| | Genagen^{®} LAA | Clariant | 3,00 % |
| | Genamin^{®} CTAC Clariant | | 1,00 % |
| | quat. Polysiloxan I | Clariant | 0,50 % |
| | Tetranatrium EDTA | | 0,10 % |
| | NIGAGUARD^{®} DMDMH | Clariant | 0,30 % |
| | Genapol^{®} PDB | Clariant | 3,00 % |
| | Kaliumphosphat | | 1,50 % |
| D | Zitronensäure | | |

### Herstellung:

- I: Lösen der Komponenten unter Rühren
- II: Mischen der Komponenten B und Erwärmen bis zur klaren Lösung
- III: Abkühlen von B auf ca. 35°C und nacheinander Zugabe der Komponenten C zu II
- IV: Einrühren von I in III
- V: Einstellen auf pH 5,5 mit D

### Beispiel 16: Haar-Gel

| | | | |
|---|---|---|---|
| A | Aristoflex^{®} AVC | Clariant | 1,40 % |
| | Wasser | | ad 100 % |
| B | Diaformer Z-751 | | 3,00 % |
| | Alkohol denat. | | 30,00 % |
| | Genapol^{®} C100 | Clariant | 0,40 % |
| | Duftstoff | | 0,20 % |
| C | Farbstoff | | q.s. |
| | Phenonip^{®} | Clariant | 0,50 % |
| D | quat. Polysiloxan I | Clariant | 0,50 % |

### Herstellung:

- I: Lösen der Komponenten A
- II: Mischen der Komponenten B
- III: Zugabe von II zu I unter Rühren
- IV: Zugabe von C zu III
- V: Zugabe von D zu IV

### Beispiel 17: Haarspitzenpflege

| | | | |
|---|---|---|---|
| A | Wasser | | 50,0 % |
| B | Tylose^{®} H 100000 G4 | | 1,00 % |
| C | Wasser | | ad 100 % |
| D | Genamin^{®} PDAC | Clariant | 2,50 % |
| | Glycerin | | 2,00 % |
| | quat. Polysiloxan I | Clariant | 1,00 % |
| E | Zitronensäure | | q.s. |

### Herstellung:

- I: B in A aufquellen
- II: Nacheinander Auflösen der einzelnen Komponenten von D in C
- III: Zugabe von II zu I
- IV: Einstellen des pH-Wertes mit E

### Beispiel 18: Syling Mousse, klar

| | | | |
|---|---|---|---|
| A | Genapol^{®} C-100 | (Clariant) | 0.60% |
| B | Wasser | | ad 100% |
| | Konservierungsmittel | | q.s. |
| C | Diaformer^{®} Z-731 | (Clariant) | 6.00% |
| | Glycerin | | 4.00% |
| | Dow Corning 193 Surfactant | | 0.30% |
| | quat. Polysiloxan I | (Clariant) | 1,25% |

### Herstellung:

- I: Mischen von A und B
- II: Nacheinander Zugabe der Komponenten C zu I

### Chem. Bezeichnung der eingesetzten Handelsprodukte

| | | |
|---|---|---|
| Aristoflex^{®} AVC | (Clariant) | Ammoniumacryloyldimethyl-Taurat/NVP Copolymer (NVP: N-Vinylpyrrolidon) |
| Aristoflex^{®} PEA 70 | (Clariant) | Polypropylenterephthalat |
| Diaformer Z-751 | | Lauryl-/Stearylacrylat, Ethylenaminoxid, Methacrylat Copolymer |
| Emulsogen^{®} HCO 040 | (Clariant) | PEG-40 Hydrogenated Castoroil |
| Eusolex 232 | (Merck) | Phenylbenzimidazolsulfonsäure |
| Genagen^{®} CAB 818 | (Clariant) | Cocoamidopropylbetain |
| Genagen^{®} KB | (Clariant) | Cocobetain |
| Genagen^{®} LAA | (Clariant) | Natriumlauroamphoacetat |
| Genamin^{®} BTLF | Clariant | Behenyltrimoniumquat |
| Genamin^{®} CTAC | (Clariant) | Cetrimoniumchlorid |
| Genamin^{®} KSL | (Clariant) | PEG-5 Stearylammoniumlactat |
| Genamin^{®} PDAC | (Clariant) | Polyquaternium-6 |
| Genapol^{®} C100 | (Clariant) | Coceth-10 |
| Genapol^{®} PDB | (Clariant) | Glycoldistearat/Laureth-4/-Cocoamidopropylbetain |
| Genapol^{®} LRO fl. | (Clariant) | Natriumlaurethsulfat |
| Genapol L3 | (Clariant) | Laureth-3 |
| Genapol PGL | (Clariant) | Glycol Distearat/ Laureth-4/. Cocoamidopropylbetain |
| Genapol^{®} T 500 P Guar | (Clariant) | Ceteareth-50 |
| Glucamat DOE-120 | | PEG-120 Methylglusosedioleat |
| Hostacerin^{®} DGI | (Clariant) | Polyglyceryl-2-Sesquiisostearat |
| Hydrolyzed silk | | hydrolysierte Aminosäure |
| NIPAGUARD DCB | (Clariant) | Phenoxyethanol, Methyldibromglutaronitril |
| NIGAGUARD^{®} DMDMH | (Clariant) | DMDM Hydantoin |
| Octopirox^{®} | (Clariant) | Pirocton Olamin |
| Phenonip^{®} | (Clariant) | Phenoxyethanol/ Methyl-/Ethyl-/-Butyl-/Propyl-/Isobutylparaben |
| Sandopan^{®} DTC, Säure | (Clariant) | Trideceth-7-Carbonsäure |
| SilCare^{®} Silicone SEA | (Clariant) | modifiziertes PolyorganosiloxanP |
| Tylose^{®} H 100000 G4 | | Hydroxyethylcellulose |
| Uvinol P 25 | (BASF) | ethoxyliertes Aminobenzoat |
| Uvinol MS 40 | (BASF) | Diethylhexylbutamidotriazol |

## Patentansprüche

1. Verwendung von mehrfach quaternären Polysiloxanen der Formel (S1) 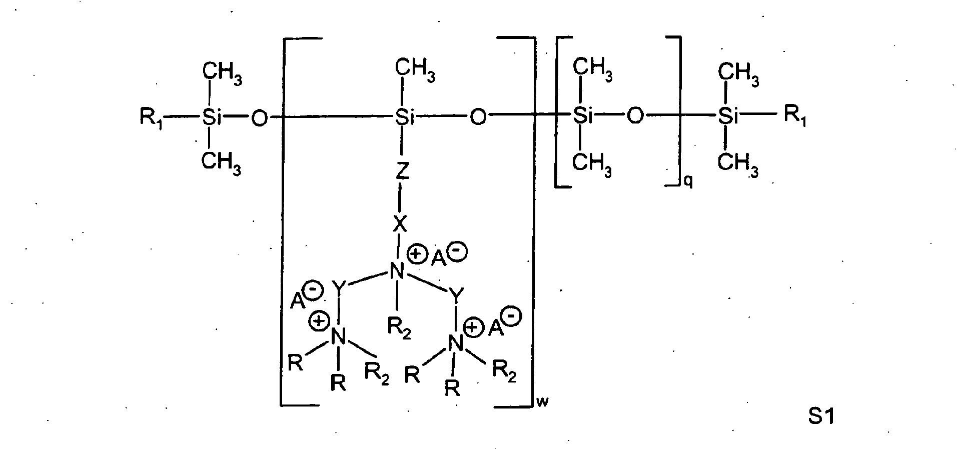 worin
die Summe aus (q + w) einen Bereich von 10 bis 1500, vorzugsweise von 15 bis 600 aufweist, und das Verhältnis q/w einen Bereich von 5 bis 600, vorzugsweise von 10 bis 400 aufweist.
R C₁-C₄-Alkyl, linear oder verzweigt, bedeutet,
R₁ Wasserstoff, C₁-C₃₋Alkyl oder C₁-C₃-Alkoxy,
R₂ C₁-C₇-Alkyl oder Benzyl,
X eine direkte Bindung,
oder 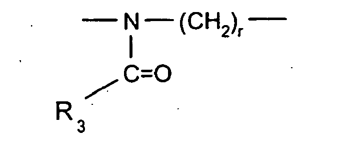 worin
r eine ganze Zahl von 1 bis 4, und
R₃ C₁-C₇-Alkyl oder -NH-C₁-C₇-Alkyl bedeutet,
oder 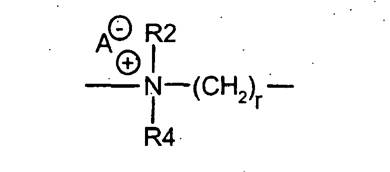 worin
R₂ und r wie vorher definiert sind und
R₄ C₁-C₃-Alkyl bedeutet,
oder 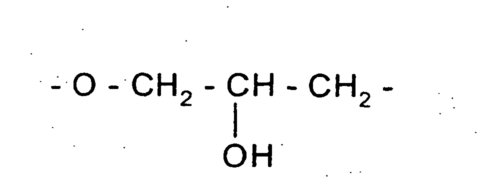 bedeutet,
Y 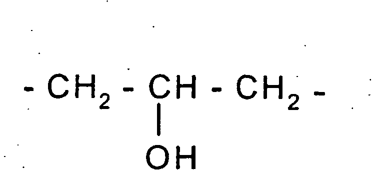
oder
-(CH₂)ₓ- bedeutet,
worin x eine ganze Zahl von 1 bis 4 darstellt,
Z C₂-C₄-Alkylen, linear oder verzweigt, ist,
A⁻ CH₃OSO₃⁻, Chlorid, Bromid, lodid oder Tosylsulfat⁻ darstellt
oder der Formel (S2) 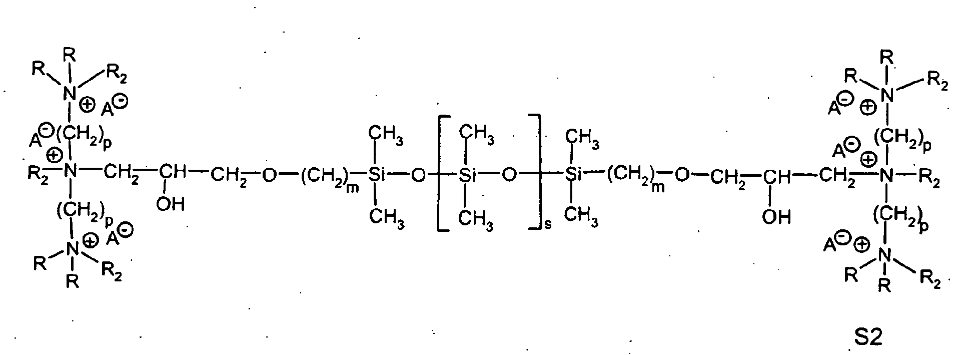 worin
R, R₂ und A⁻ die gleiche Bedeutung wie in Formel (S1) haben,
m eine ganze Zahl von 1 bis 4,
p eine ganze Zahl von 1 bis 4, und
s eine Zahl im Bereich von 5 bis 1500, vorzugsweise von 10 bis 600 bedeuten,
in kosmetischen oder pharmazeutischen Mitteln zum Reinigen und Pflegen keratinischer Fasern, insbesondere von menschlichen Haaren.

2. Verwendung gemäß Patentanspruch 1, **dadurch gekenntzeichnet, dass** eines oder mehrere der Polysiloxane enthaltend die Struktureinheit E1 bis E6 eingesetzt werden

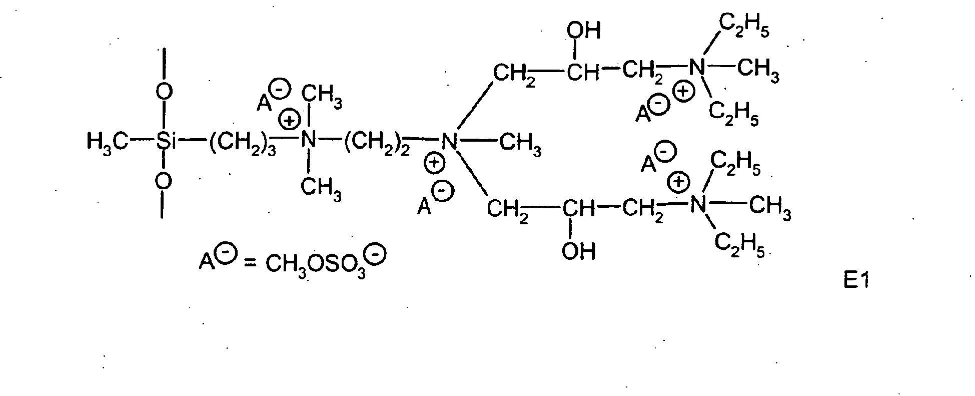

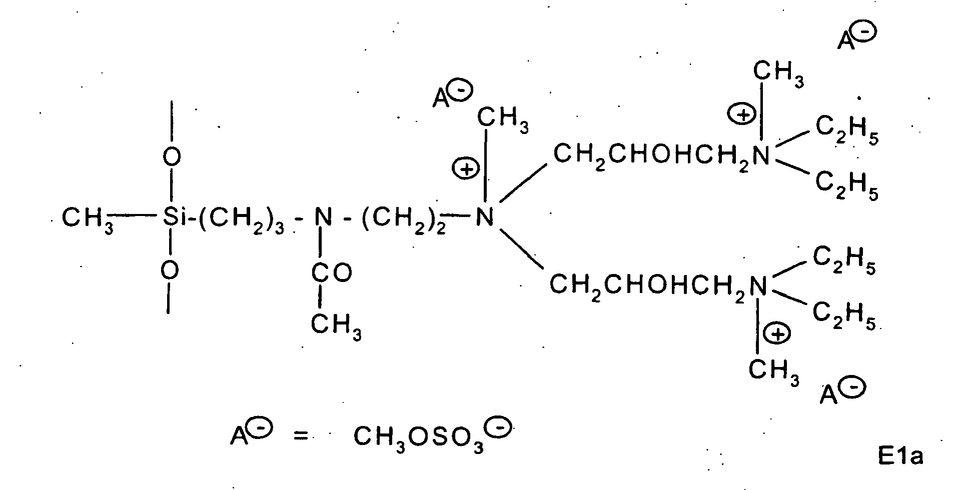

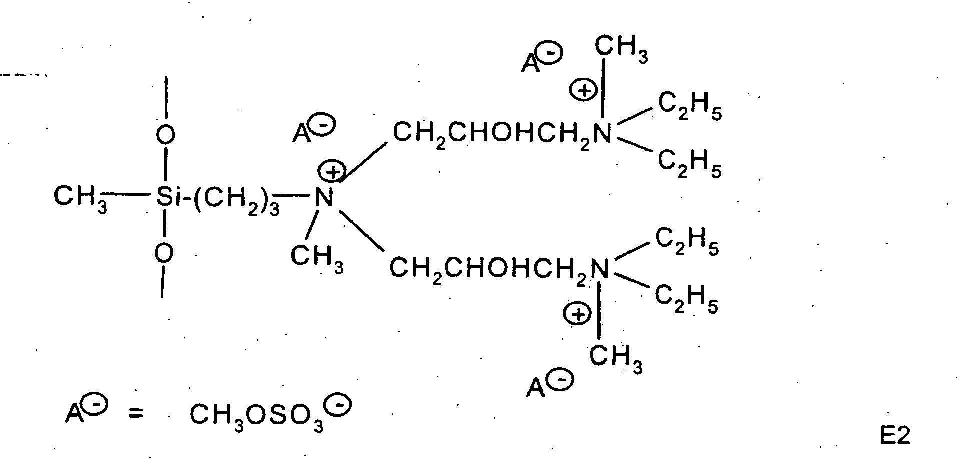

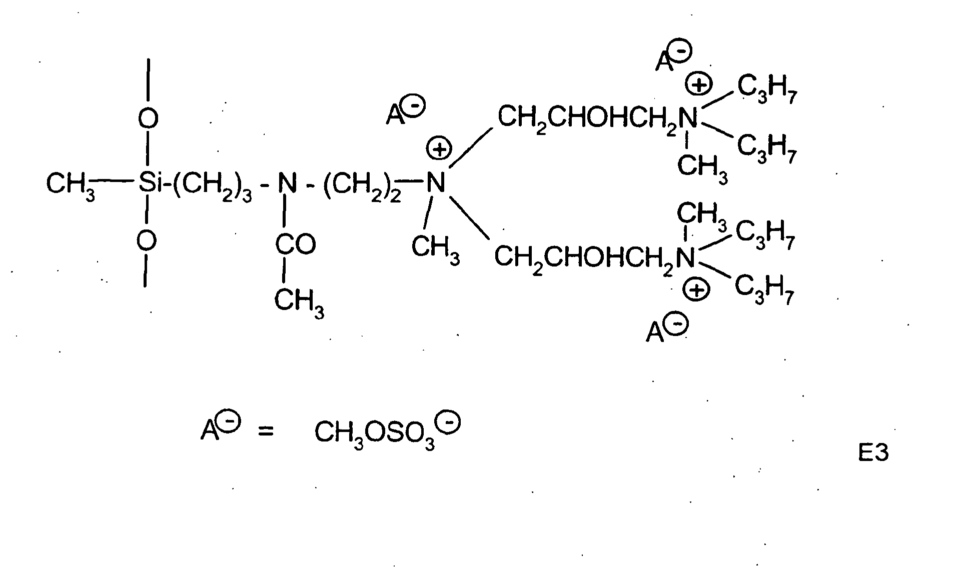

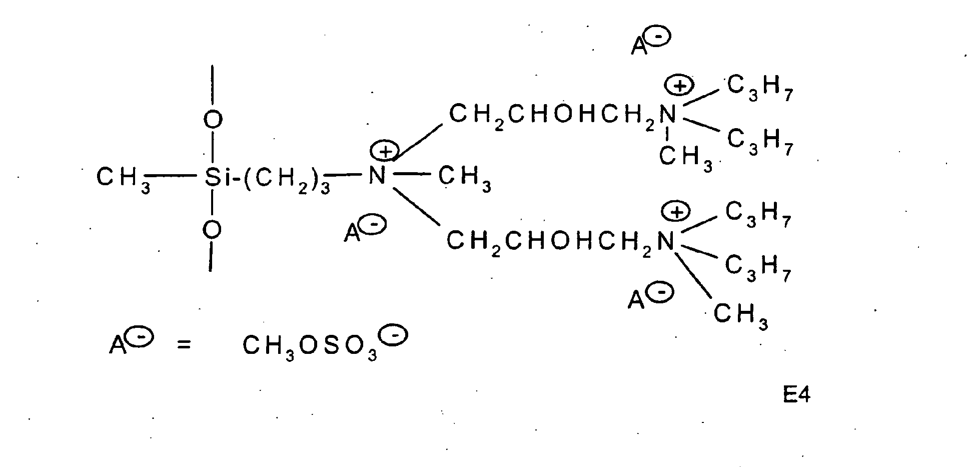

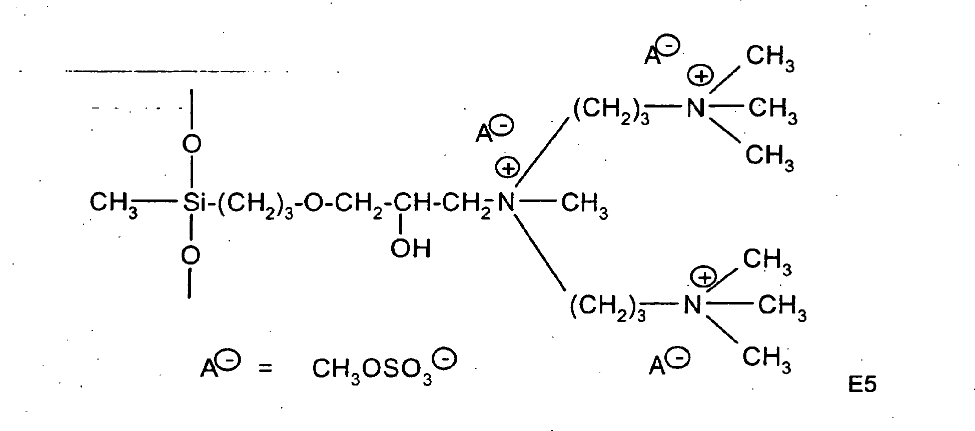

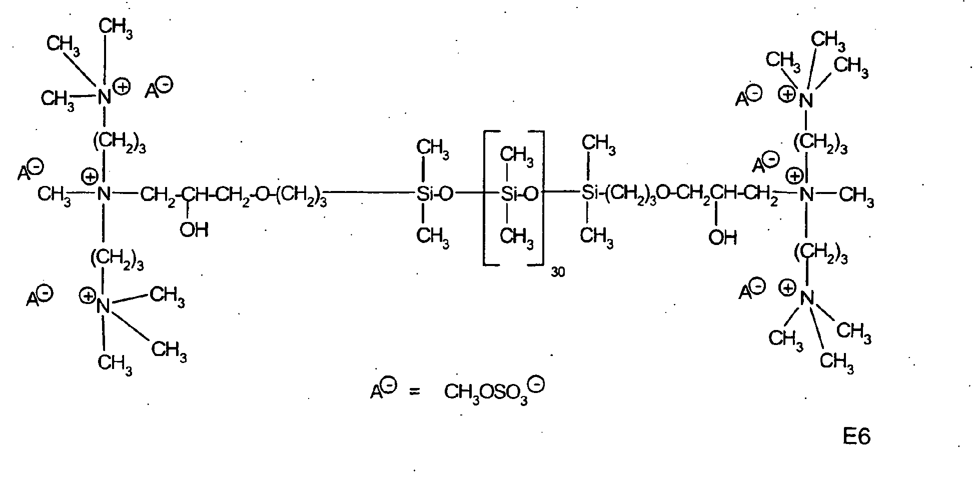

3. Verwendung gemäß Patentanspruch 1, **dadurch gekennzeichnet, dass** eines oder mehrere der Polysiloxane enthaltend die Struktureinheit E1 eingesetzt werden 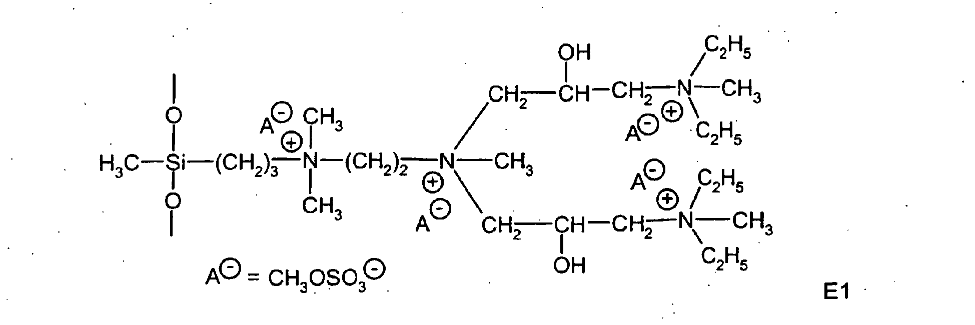
wobei A⁻ CH₃OSO₃⁻, Chlorid, Bromid, lodid oder Tosylsulfat⁻ darstellt.

4. Verwendung nach einem oder nach mehreren der Ansprüche 1 bis 3, **dadurch gekennzeichnet, dass** die Polysiloxane in den kosmetischen oder pharmazeutischen Mitteln in wässriger, wässrig-alkoholischer, alkoholischer oder wässrig-tensidischer Form vorliegen oder dass sie Mittel auf Basis von Öl darstellen oder dass sie als Emulsion, Suspension oder Dispersion vorliegen, vorzugsweise in Form von Fluids, Schäumen, Sprays, Gelen, Mousse, Lotionen oder Cremes.

5. Verwendung nach einem oder nach mehreren der Ansprüche 1 bis 4, **dadurch gekennzeichnet, dass** es sich bei den kosmetischen und pharmazeutischen Mitteln um tensidfreie Mittel, um tensidfreie Emulsionen, Gele, Sprays, Sprühschäume, Mousse oder Fluids handelt.

6. Verwendung nach einem oder nach mehreren der Ansprüche 1 bis 5, **dadurch gekennzeichnet, dass** es sich bei den kosmetischen oder pharmazeutischen Mitteln um Öl-in-Wasser Emulsionen mit einem Wasseranteil von 5 bis 95 Gew.-%, bevorzugt 15 bis 75 Gew.-%, besonders bevorzugt 25 bis 85 Gew.-% handelt.

7. Verwendung nach einem oder nach mehreren der Ansprüche 1 bis 5, **dadurch gekennzeichnet, dass** es sich bei den kosmetischen oder pharmazeutischen Mitteln um Mittel auf wässrig-alkoholischer oder alkoholischer Basis handelt, die Alkohole mit 1 bis 4 Kohlenstoffatomen wie Ethanol, Propanol, Isopropanol, n-Butanol, i-Butanol, t-Butanol oder Glycerin sowie Alkylenglykole, insbesondere Propylen-, Butylen- oder Hexylenglykol, oder Mischungen aus den genannten Alkoholen enthalten oder Polyethylenglykole mit einer relativen Molekülmasse unter 2000.

8. Verwendung nach einem oder nach mehreren der Ansprüche 1 bis 7, **dadurch gekennzeichnet, dass** es sich bei den kosmetischen oder pharmazeutischen Mitteln um Additive für Haarpflegemittel, insbesondere zur Verbesserung des Glanzes und des Farbschutzes der Haare handelt.

9. Verwendung nach einem oder nach mehreren der Ansprüche 1 bis 8, **dadurch gekennzeichnet, dass** die Polysiloxane in den kosmetischen oder pharmazeutischen Mitteln in einer Menge im Bereich von 0,01 bis 30 %, bevorzugt von 0,2 bis 10 %, besonders bevorzugt von 0,5 bis 2 %, bezogen auf die fertigen Mittel, eingesetzt werden.

## Claims

1. The use of polyquaternary polysiloxanes of the formula (S1) 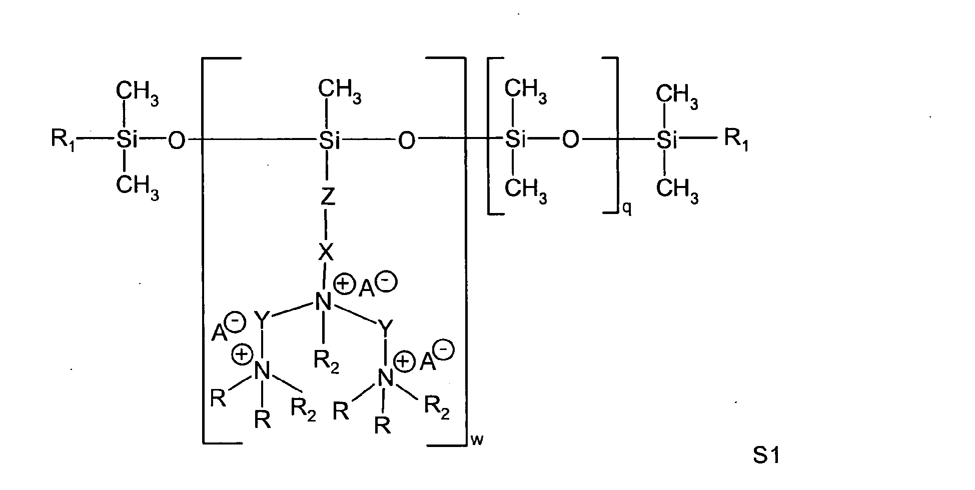 in which
the sum of (q + w) has a range from 10 to 1500, preferably from 15 to 600, and the ratio q/w has a range from 5 to 600, preferably from 10 to 400,
R is C₁-C₄-alkyl, linear or branched,
R₁ is hydrogen, C₁-C₃-alkyl or C₁-C₃-alkoxy,
R₂ is C₁-C₇-alkyl or benzyl,
X is a direct bond,
or 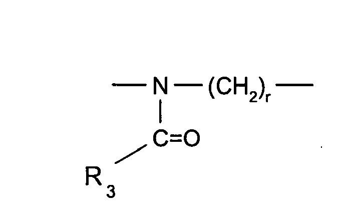 in which
r is an integer from 1 to 4, and
R₃ is C₁-C₇-alkyl or -NH-C₁-C₇-alkyl,
or 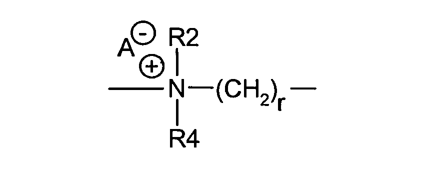 in which
R₂ and r are as defined previously and
R₄ is C₁-C₃-alkyl,
or 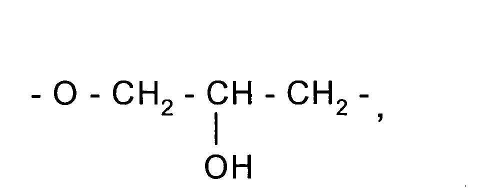
Y is 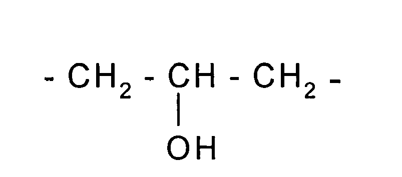
or
-(CH₂)ₓ-,
in which x is an integer from 1 to 4,
Z is C₂-C₄-alkylene, linear or branched,
A⁻ is CH₃OSO₃⁻, chloride, bromide, iodide or tosylsulfate⁻
or of the formula (S2) 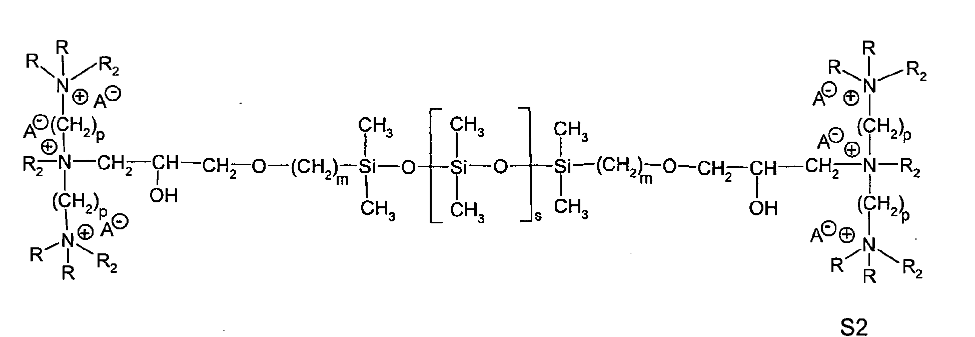 in which
R, R₂ and A⁻ have the same meaning as in formula (S1),
m is an integer from 1 to 4,
p is an integer from 1 to 4, and
s is a number in the range from 5 to 1500, preferably from 10 to 600,
in cosmetic or pharmaceutical compositions for the cleaning and care of keratin fibers, in particular of human hair.

2. The use as claimed in patent claim 1, wherein one or more of the polysiloxanes comprising the structural unit E1 to E6 are used

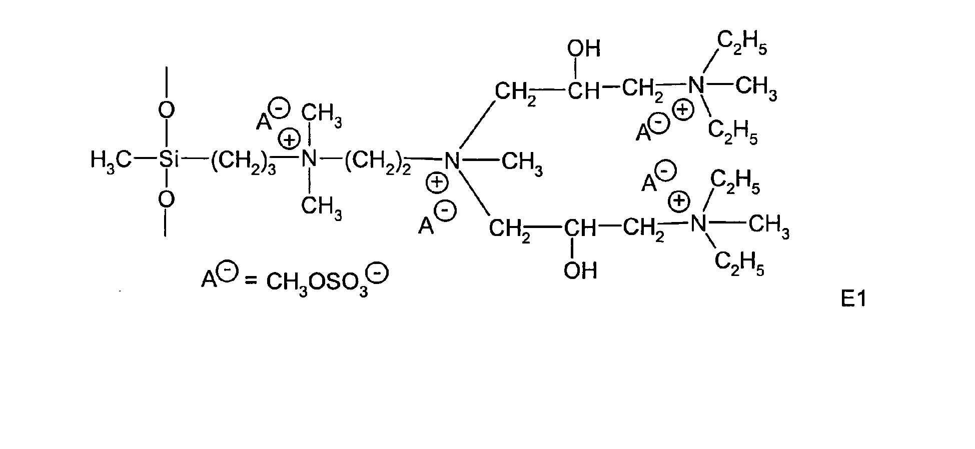

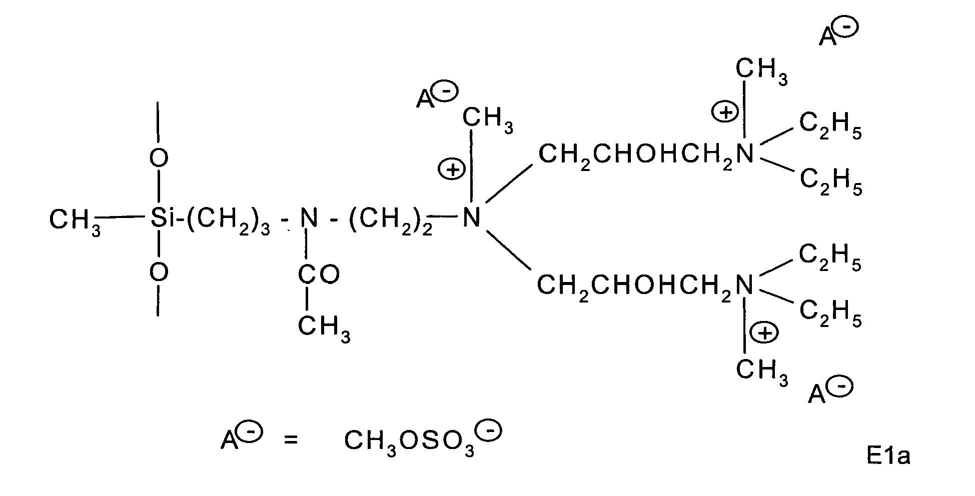

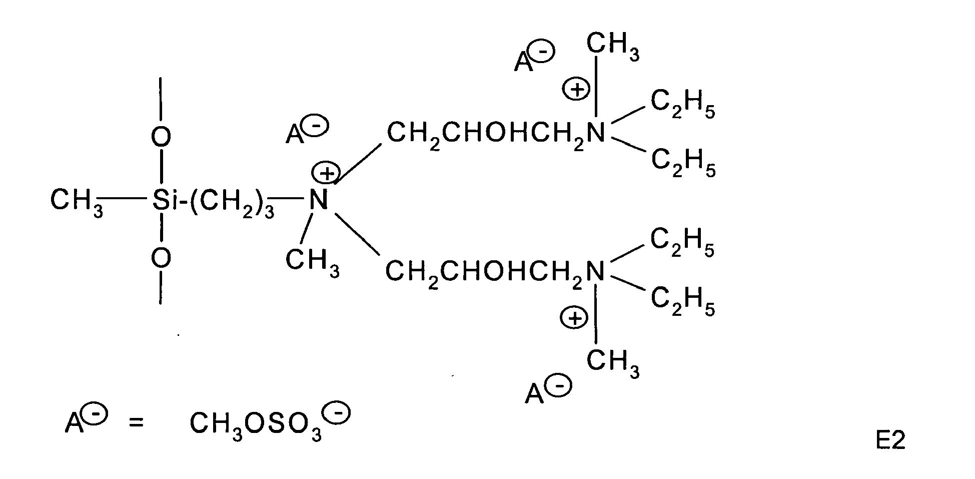

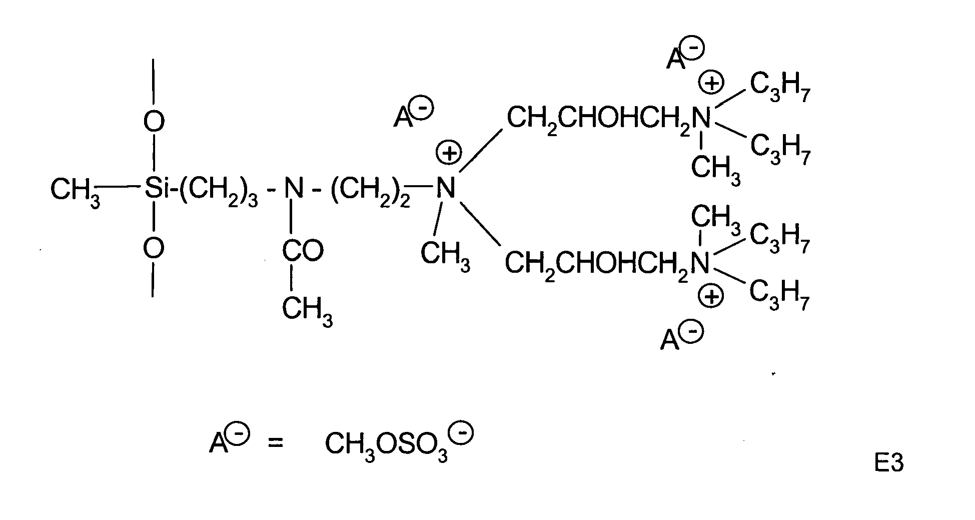

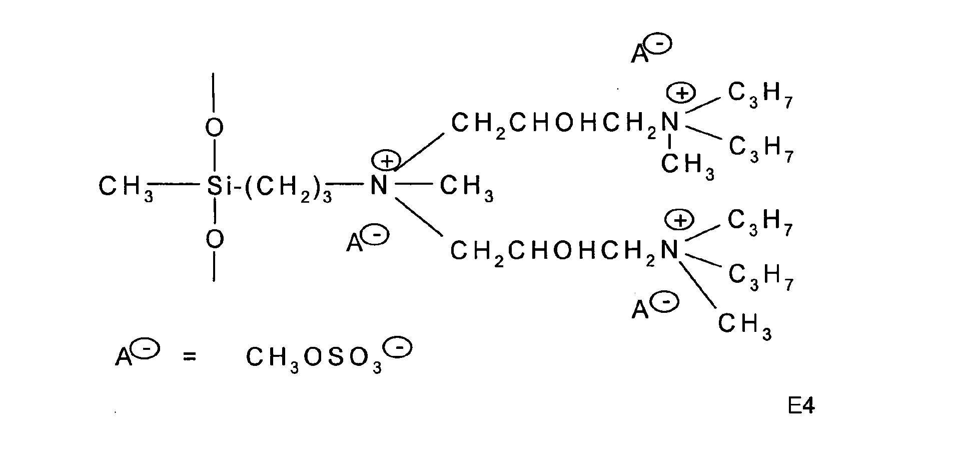

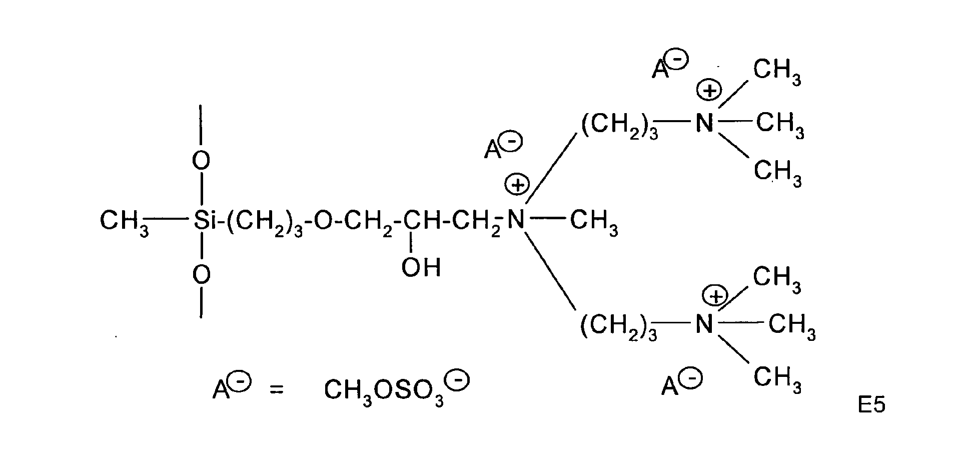

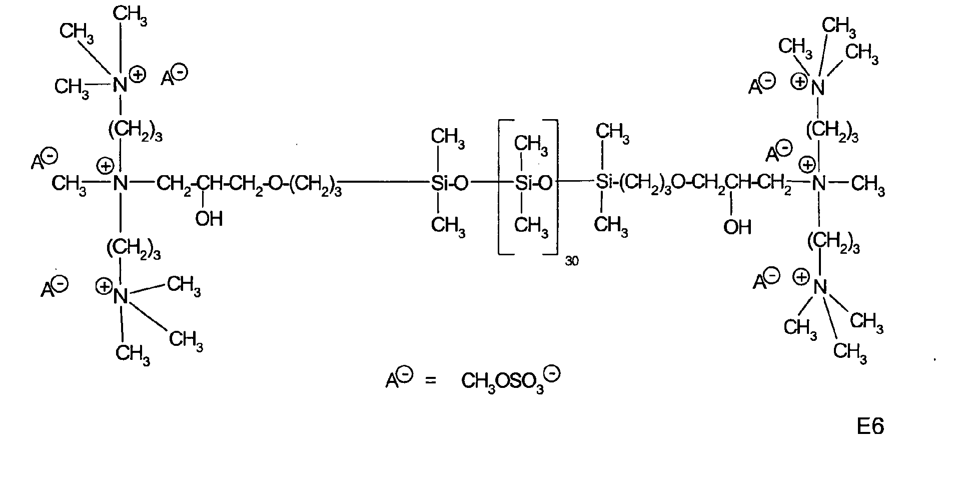

3. The use as claimed in patent claim 1, wherein one or more of the polysiloxanes comprising the structural unit E1 are used 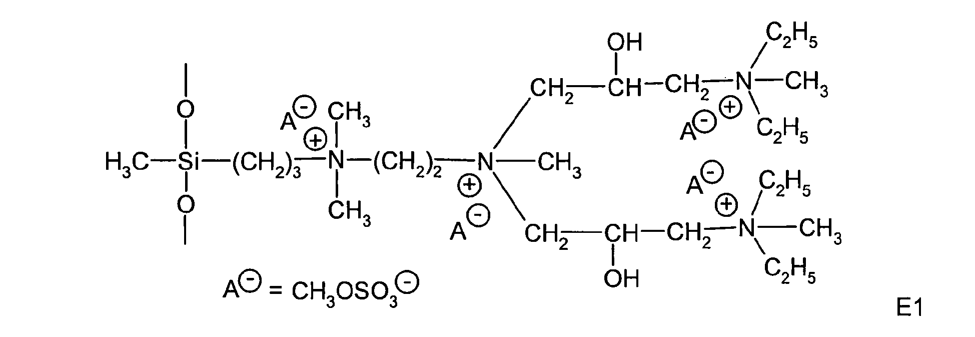
where A⁻ is CH₃OSO₃⁻, chloride, bromide, iodide or tosylsulfate⁻.

4. The use as claimed in one or more of claims 1 to 3, wherein the polysiloxanes are present in the cosmetic or pharmaceutical compositions in aqueous, aqueous-alcoholic, alcoholic or aqueous-surface-active form, or they are compositions based on oil, or they are in emulsion, suspension or dispersion form, preferably in the form of fluids, foams, sprays, gels, mousse, lotions or creams.

5. The use as claimed in one or more of claims 1 to 4, wherein the cosmetic and pharmaceutical compositions are surfactant-free compositions, surfactant-free emulsions, gels, sprays, spray foams, mousse or fluids.

6. The use as claimed in one or more of claims 1 to 5, wherein the cosmetic or pharmaceutical compositions are oil-in-water emulsions with a water fraction of from 5 to 95% by weight, preferably 15 to 75% by weight, particularly preferably 25 to 85% by weight.

7. The use as claimed in one or more of claims 1 to 5, wherein the cosmetic or pharmaceutical compositions are compositions on an aqueous-alcoholic or alcoholic basis which comprise alcohols having 1 to 4 carbon atoms, such as ethanol, propanol, isopropanol, n-butanol, isobutanol, t-butanol or glycerol, and alkylene glycols, in particular propylene glycol, butylene glycol or hexylene glycol, or mixtures of said alcohols, or polyethylene glycols with a relative molecular mass below 2000.

8. The use as claimed in one or more of claims 1 to 7, wherein the cosmetic or pharmaceutical compositions are additives for haircare compositions, in particular for improving the shine and color protection of the hair.

9. The use as claimed in one or more of claims 1 to 8, wherein the polysiloxanes are used in the cosmetic or pharmaceutical compositions in an amount in the range from 0.01 to 30%, preferably from 0.2 to 10%, particularly preferably from 0.5 to 2%, based on the finished compositions.

## Revendications

1. Utilisation de polysiloxanes polyquaternaires de formule (S1) 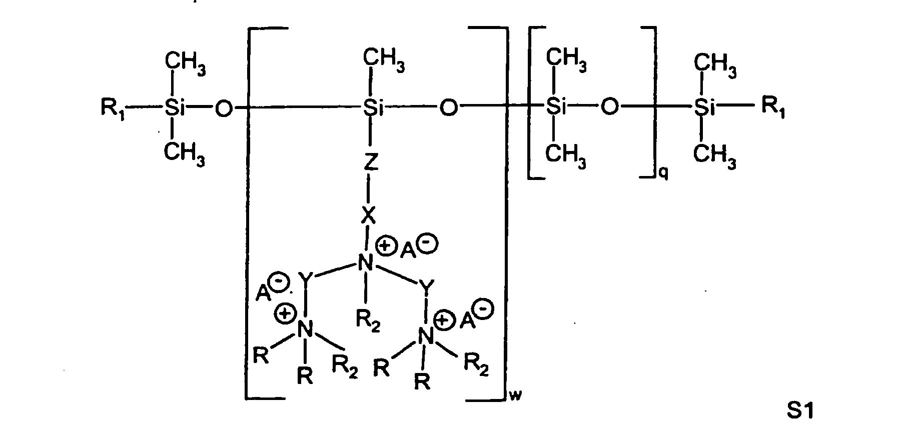 où
la somme de (q + w) présente une plage de 10 à 1500, de préférence de 15 à 600, et le rapport q/w présente une plage de 5 à 600, de préférence de 10 à 400,
R signifie C₁-C₄-alkyle, linéaire ou ramifié,
R₁ signifie hydrogène, C₁-C₃-alkyle ou C₁-C₃-alcoxy,
R₂ signifie C₁-C₇-alkyle ou benzyle,
X signifie une liaison directe,
ou 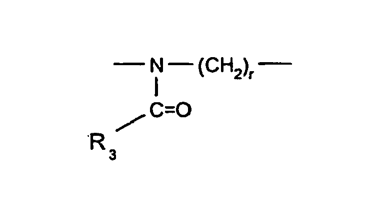 où
r vaut un nombre entier de 1 à 4, et
R₃ signifie C₁-C₇-alkyle ou -NH-C₁-C₇-alkyle,
ou 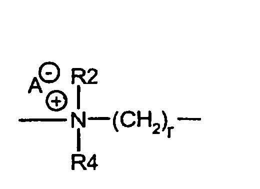 où
R₂ et r sont définis comme ci-dessus et
R₄ signifie C₁-C₃-alkyle,
ou 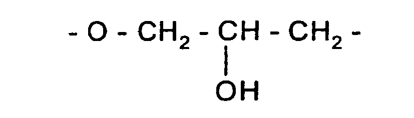
Y signifie 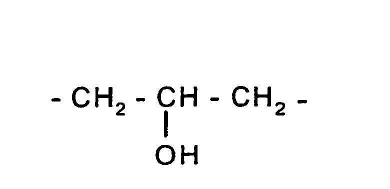
ou
**-(CH₂)ₓ-**
où x représente un nombre entier de 1 à 4,
Z représente C₂-C₄-alkylène, linéaire ou ramifié,
A⁻ représente CH₃OSO₃⁻, chlorure, bromure, iodure ou tosylsulfate⁻
ou de formule (S2) 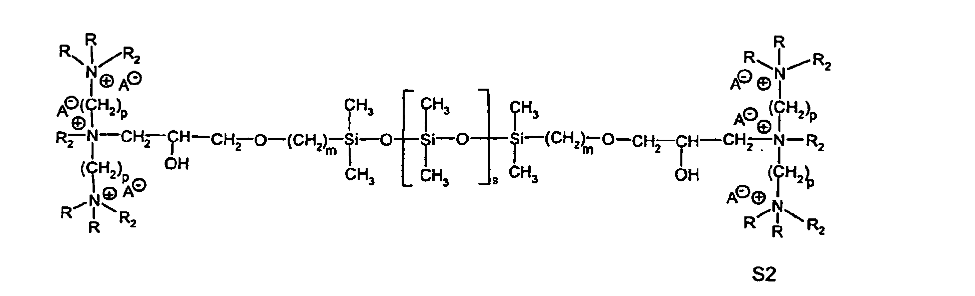 où
R, R₂ et A⁻ ont la même signification que dans la formule (S1),
m vaut un nombre entier de 1 à 4,
p vaut un nombre entier de 1 à 4, et
s signifie un nombre dans la plage de 5 à 1500, de préférence de 10 à 600,
dans des agents cosmétiques ou pharmaceutiques pour le nettoyage et les soins de fibres kératiniques, en particulier de cheveux.

2. Utilisation selon la revendication 1, **caractérisée**
**en ce qu'**on utilise un ou plusieurs des polysiloxanes contenant l'unité de structure E1 à E6

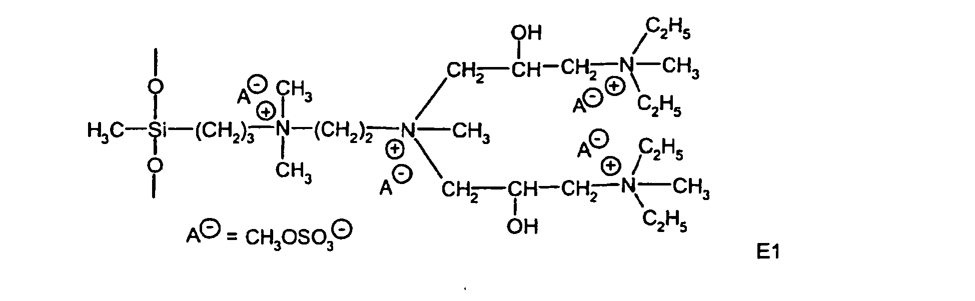

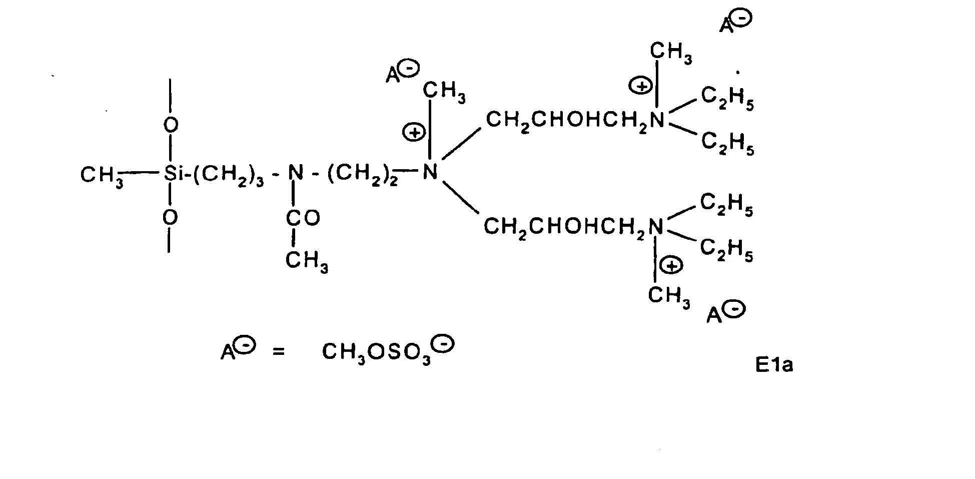

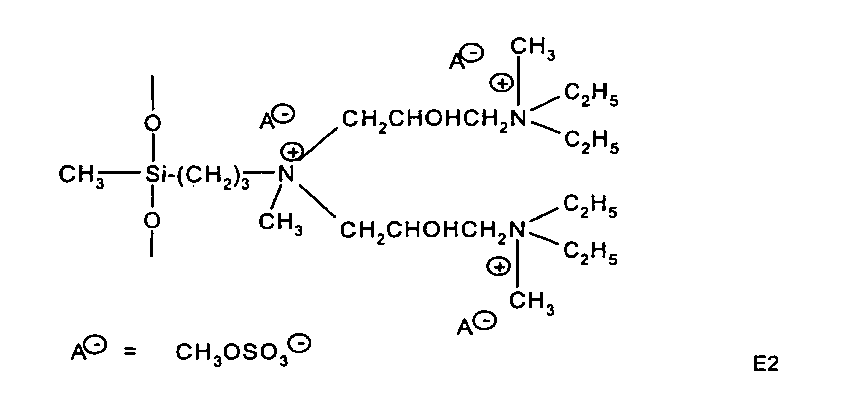

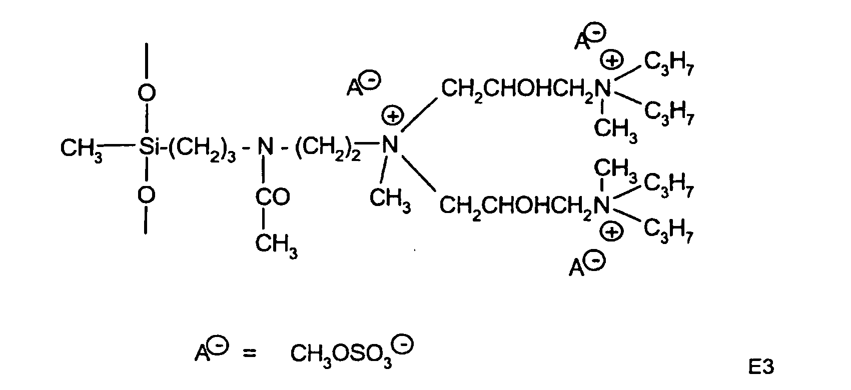

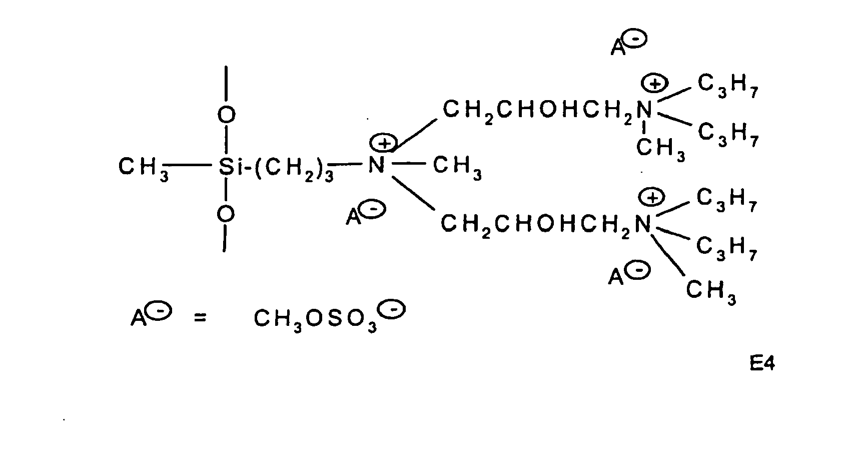

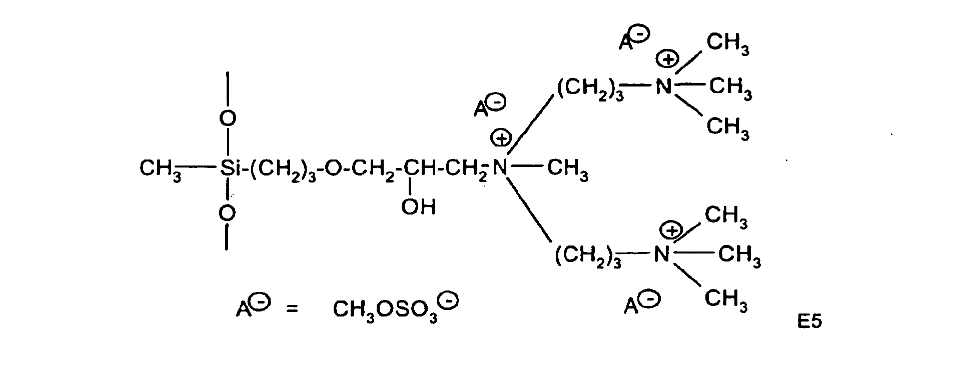

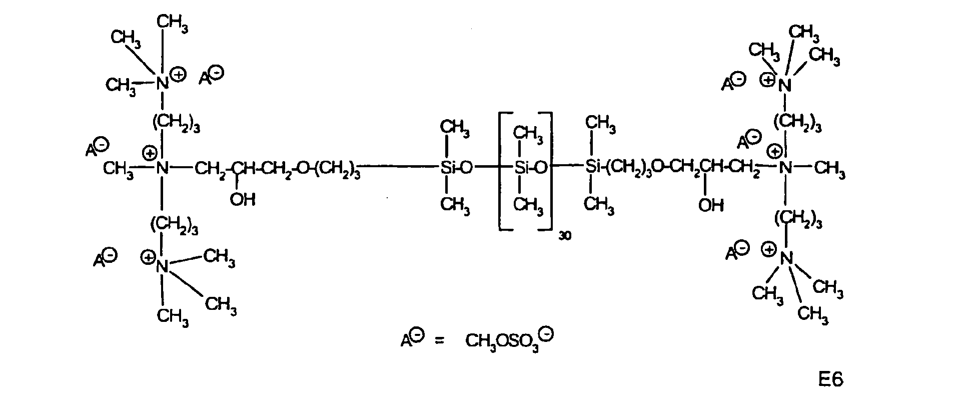

3. Utilisation selon la revendication 1, 5 **caractérisée**
**en ce qu'**on utilise un ou plusieurs des polysiloxanes contenant l'unité de structure E1 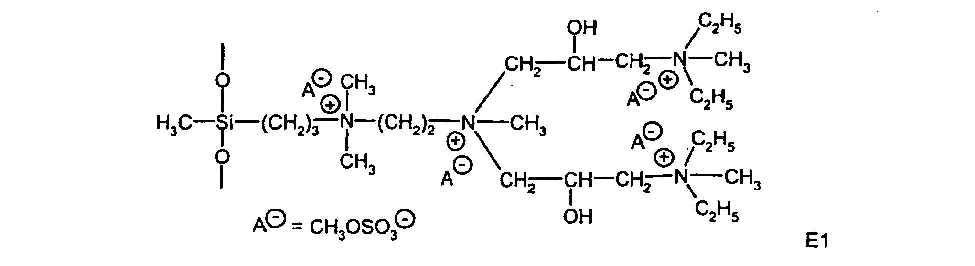 où A⁻ représente CH₃OSO₃⁻, chlorure, bromure, iodure ou 10 tosylsulfate⁻.

4. Utilisation selon l'une ou plusieurs des revendications 1 à 3, **caractérisée**
**en ce que** les polysiloxanes se trouvent dans les agents cosmétiques ou pharmaceutiques sous une forme aqueuse, aqueuse-alcoolique, alcoolique ou aqueuse-tensioactive et en ce qu'il s'agit d'agents à base d'huile ou en ce qu'ils se trouvent sous forme d'émulsion, de suspension ou de dispersion, de préférence sous forme de fluides, de mousses, de sprays, de gels, de mousse, de lotions ou de crèmes.

5. Utilisation selon l'une ou plusieurs des revendications 1 à 4, **caractérisée**
**en ce qu'**il s'agit, pour les agents cosmétiques et pharmaceutiques, d'agents exempts d'agents tensioactifs, d'émulsions, de gels, de sprays, de mousses à pulvériser, de mousses ou de fluides exempt(e)s d'agents tensioactifs.

6. Utilisation selon l'une ou plusieurs des revendications 1 à 5, **caractérisée**
**en ce qu'**il s'agit, pour les agents cosmétiques ou pharmaceutiques, d'émulsions huile-dans-eau présentant une proportion d'eau de 5 à 95% en poids, de préférence de 15 à 75% en poids, de manière particulièrement préférée de 25 à 85% en poids.

7. Utilisation selon l'une ou plusieurs des revendications 1 à 5, **caractérisée**
**en ce qu'**il s'agit, pour les agents cosmétiques ou pharmaceutiques, d'agents à base aqueuse-alcoolique ou à base alcoolique, qui contiennent des alcools comprenant 1 à 4 atomes de carbone tels que l'éthanol, le propanol, l'isopropanol, le n-butanol, l'i-butanol, le t-butanol ou le glycérol ainsi que des alkylèneglycols, en particulier le propylèneglycol, le butylèneglycol ou l'hexylèneglycol, ou des mélanges des alcools mentionnés ou des polyéthylèneglycols présentant une masse moléculaire relative inférieure à 2000.

8. Utilisation selon l'une ou plusieurs des revendications 1 à 7, **caractérisée**
**en ce qu'**il s'agit, pour les agents cosmétiques ou pharmaceutiques, d'additifs pour agents de soins des cheveux, en particulier pour améliorer la brillance et la protection de la couleur des cheveux.

9. Utilisation selon l'une ou plusieurs des revendications 1 à 8, **caractérisée**
**en ce que** les polysiloxanes sont utilisés dans les agents cosmétiques ou pharmaceutiques en une quantité dans la plage de 0,01 à 30%, de préférence de 0,2 à 10%, de manière particulièrement préférée de 0,5 à 2%, par ra à l'agent fini.
